Europäisches Patentamt

(19) European Patent Office

Office européen des brevets

(11) **EP 0 629 124 B1**

(12) **EUROPEAN PATENT SPECIFICATION**

(45) Date of publication and mention
of the grant of the patent:
**08.07.1998 Bulletin 1998/28**

(21) Application number: 93905229.6

(22) Date of filing: 26.02.1993

(51) Int. Cl.$^6$: **A61B 5/103**, G01J 3/50

(86) International application number:
**PCT/DK93/00072**

(87) International publication number:
**WO 93/16635 (02.09.1993 Gazette 1993/21)**

(54) **A METHOD AND AN APPARATUS FOR DETERMINING AN INDIVIDUAL'S ABILITY TO STAND EXPOSURE TO ULTRAVIOLET RADIATION**

VERFAHREN UND VORRICHTUNG ZUR BESTIMMUNG DER WIDERSTANDSFÄHIGKEIT VON PRÜFPERSONEN GEGENÜBER UV-STRAHLUNG

PROCEDE ET APPAREIL POUR DETERMINER LA CAPACITE D'UN INDIVIDU A RESISTER A UNE EXPOSITION A DES RADIATIONS ULTRAVIOLETTES

(84) Designated Contracting States:
**CH DE DK FR GB LI NL**

(30) Priority: 28.02.1992 DK 26592

(43) Date of publication of application:
**21.12.1994 Bulletin 1994/51**

(73) Proprietor: **CHROMO-LIGHT APS**
**DK-2920 Charlottenlund (DK)**

(72) Inventor: **WULF, Hans, Christian**
**DK-2920 Charlottenlund (DK)**

(74) Representative:
**Nielsen, Henrik Sten et al**
**OSTENFELD PATENTBUREAU A/S,**
**Bredgade 41,**
**P.O. Box 1183**
**1011 Copenhagen K (DK)**

(56) References cited:

| | |
|---|---|
| WO-A-88/05284 | WO-A-91/14159 |
| US-A- 4 423 736 | US-A- 4 749 865 |
| US-A- 4 846 184 | US-A- 4 882 598 |

Printed by Xerox (UK) Business Services
2.16.3/3.4

## Description

The present invention relates to the technique of determining an individual's ability to become tanned or to stand exposure to ultraviolet radiation without causing a skin reaction, such as skin cancer or erythema, which technique is described in US Patent No. 4.882.598, to which US Patent reference is made.

According to the technique described in the above US Patent, the individual's ability to become tanned or to stand exposure to ultraviolet radiation without causing a skin reaction may be determined by exposing a skin surface part of the individual to electromagnetic radiation, e.g. visible light, and determining the coefficient of reflection of the individual's skin surface part to the electromagnetic radiation and converting the coefficient of reflection to a measure representing the individual's ability to become tanned or to stand exposure to ultraviolet radiation. The technique described in the above US Patent is based on clinical experiments revealing a linear relationship between a number of individuals coefficients of skin surface reflection in logarithmic representation and the same individuals' ability to stand exposure to ultraviolet radiation of a predetermined intensity and of a predetermined spectral composition. According to the above-mentioned US Patent, individuals who are erythrodermic or erythematous may further be identified by performing the determination of the individuals' coefficient of reflection to electromagnetic radiation at two distinct wavelengths, one of which is a wavelength at which erythrodermic skin reflection is high, and another one of which is a wavelength at which erythrodermic skin reflection is low.

The basis of the present invention is the realization that the determination of the individual's coefficient of reflection or reflections to electromagnetic radiation is to a high degree influenced by the blood flow of the individual at the site or the skin surface part at which the measurement is carried out. An obvious solution solving the problem of eliminating the blood flow influence on the determination of the measure representing the individual's ability to become tanned or to stand exposure to ultraviolet radiation in accordance with the teaching of the above US Patent is to specify a "normal" measuring routine involving that the individual is kept in a normalized position and state, e.g. specifying the site or the part of the individual's skin surface at which the measurement is to be performed, the position of the site or skin surface part in question, and the position of the individual and further a specific increased or reduced blood flow accomplished through an occlusion of a body part of the individual, on which body part the site or skin surface part is positioned, or alternatively a state of extreme blood flow, e.g. provoked through heating the site or the skin surface part at which the measurement is to be performed. In numerous instances, the individuals cannot be maintained in a normalized position and state corresponding to the above specified circumstances or similar relevant specified circumstances for numerous reasons. Also, a so-called normalized state may often result in an extreme variation of the measuring results obtained, which makes the overall measuring technique unreliable and inadequate.

An object of the present invention is to provide a method of determining an individual's ability to become tanned or to stand exposure to ultraviolet radiation without causing a skin reaction, which method is independent of the blood flow of the individual, and which method renders it possible to provide a measure representing said individual's ability independent on the blood flow of the individual.

A further object of the present invention is to provide a method of determining an individual's ability to become tanned or to stand exposure to ultraviolet radiation, which method produces normalized measuring results which are readily comparable, rendering it possible to obtain a high degree of reproducibility of the determination.

A further object of the present invention is to provide an apparatus for determining an individual's ability to become tanned or to stand exposure to ultraviolet radiation, which apparatus is capable to perform a measuring routine and generate a measuring result independent of the blood flow of the individual, the ability of which is to be determined.

A still further object of the present invention is to provide an apparatus of the above type of an extreme reliability and producing measuring results of high reproducibility and high accuracy.

According to a first aspect of the present invention, a method of determining an individual's ability to become tanned or to stand exposure to ultraviolet radiation without causing a skin reaction, such as skin cancer or erythema, comprising the following steps:

exposing at least part of said individual's skin surface to electromagnetic radiation of a first wavelength and of a predetermined intensity, said first wavelength being a wavelength at which erythrodermic skin reflection is high,

measuring the intensity of electromagnetic radiation reflected from said part of said individual's skin surface so as to determine a first coefficient of reflection of said skin surface part to said electromagnetic radiation of said first wavelength,

exposing said skin surface part to electromagnetic radiation of a second wavelength and of a predetermined intensity, said second wavelength being a wavelength at which erythrodermic skin reflection is low,

measuring the intensity of electromagnetic radiation reflected from said part of said individual's skin surface part so as to determine a second coefficient of reflection of said skin surface part to electromagnetic radiation of said second wavelength,

comparing said first and second coefficients of reflection with predetermined sets of coefficients of reflection representing coherent sets of coefficients of reflection of said first and second wavelengths of specific states of redness so as to determine said individual's skin surface part's state of redness, converting said first and second coefficients of reflection into a set of corrected first and second coefficients of reflection of a specific state of redness, so as to determine said individual's skin surface part's coefficients of reflection of said first and second wavelengths at a specific state of redness, and

converting said corrected first coefficient of reflection into a measure representing said individual's ability to become tanned or to stand exposure to ultraviolet radiation without causing said skin reaction.

Basically, in accordance with the teaching of the present invention, it has been realized through clinical investigations that the first and the second coefficients of reflection determined in accordance with the teaching of the above US Patent may be converted to a set of corrected first and second coefficients of reflection of a specific state of redness since the first and the second coefficients of reflection at different states of redness, and consequently different blood flows, fulfil specific mathematic relations, as will be evident from the description below, rendering it possible to convert a set of first and second coefficients of reflection to a set of corrected first and second coefficients of reflection of a specific state of redness. Preferably, according to the teaching of the present invention, the specific state of redness to which the first and second coefficients of reflections measured in accordance with the method according to the present invention and in accordance with the teaching of the above US Patent corresponds to an average zero blood flow state, i.e. a state in which the individual's blood flow is zero or extremely low, eliminating to any substantial extent the influence of the redness of the individual on the coefficients of reflection.

According to a further realization according to the teaching of the present invention, the method advantageously further comprises the step of determining the individual's skin surface part's degree of pigmentation from the corrected first and second coefficients of reflection of the first and second wavelengths at the specific state of redness.

The basic clinical investigations on which the present invention is based have revealed that provided the coefficient of reflection of said second wavelength is represented in logarithmic representation, the mathematic relation through which sets of coefficients of reflection representing coherent sets of coefficients of reflection of said first and second wavelengths are linearly related, rendering the conversion of the first and the second coefficients of reflection into a set of coherent first and second coefficients of reflection extremely simple and readily adaptable to automatized conversion, e.g. by means of a computer, such as a microprocessor.

In accordance with a second aspect of the present invention, an apparatus is provided for determining an individual's ability to become tanned or to stand exposure to ultraviolet radiation without causing a skin reaction, such as skin cancer or erythema, comprising:

a first electromagnetic source for generating electromagnetic radiation of a first wavelength and of a predetermined intensity and for directing said electromagnetic radiation of said first wavelength to a part of said individual's skin surface so as to expose said part of said individual's skin surface to said electromagnetic radiation of said first wavelength,

a second electromagnetic source for generating electromagnetic radiation of a second wavelength and of a predetermined intensity and for directing said electromagnetic radiation of said second wavelength to said part of said individual's skin surface so as to expose said part of said individual's skin surface to said electromagnetic radiation of said second wavelength,

a light-detecting means for measuring the intensity of electromagnetic radiation reflected from said part of said individual's skin surface,

a measuring means connected to said light-detecting means for measuring the intensity of electromagnetic radiation reflected from said part of said individual's skin surface so as to determine a first and a second coefficient of reflection of said skin surface part to said electromagnetic radiation of said first and second wavelength, respectively,

a comparison and converting means connected to said measuring means for comparing said first and second coefficients of reflection with predetermined sets of coefficients of reflection representing coherent sets of coefficients of reflection of said first and second wavelengths of specific states of redness so as to determine said individual's skin surface part's state of redness, for converting said first and second coefficients of reflection into a set of corrected first and second coefficients of reflection of a specific state of redness, so as to determine said individual's skin surface part's coefficients of reflection of said first and second wavelengths at a specific state of redness, and for converting said corrected first coefficient of reflection into a measure representing said individual's ability to become tanned or to stand exposure to ultraviolet radiation without causing said skin reaction.

Basically, the apparatus according to the present invention may be implemented in accordance with the above embodiments of the method according to the present invention. Furthermore, according to a first embodiment of the apparatus, separate light detector means for detecting electromagnetic radiation of the first and the second wavelengths, respectively, may be provided. Preferably, the apparatus according to the present invention, however, comprises a single light-detecting means constituted by a single light detector, first of all simplifying the structure of the apparatus and secondly ensuring that the reflection of light of the first and the second wavelength is generated at one and the same part of the individual's skin surface and further eliminates any difference in sensitivity and consequently measuring accuracy between the measurement of the reflection of electromagnetic radiation of the first and the second wavelength.

Due to the high accuracy of the measuring technique according to the present invention, the UV treatment of e.g. psoriasis patients may be adapted so as to carry out a full-body scanning of the body of the patient for carrying out an optimum UV treatment of the entire body of the patient, at which treatment, any differences as to UV sensitivity of different skin surface areas of the patient are complied with, so that, independent of the difference in UV sensitivity of the differnet skin surface areas of the patient, any skin surface area of the patient is exposed to an optimum UV treatment.

The present invention will now be further described with reference to the drawings, in which

Fig. 1 is a diagram illustrating the correspondence between the coefficient of skin surface reflection and the wavelength of the electromagnetic radiation, to which the skin surface part has been exposed, further illustrating a curve A illustrating the response of an average, non-sun-tanned and non-erythrodermic skin surface part of an individual and a curve B illustrating the response of an erythrodermic or sun-burnt skin surface part of an individual,

Fig. 2 is a diagram illustrating the correspondence between the coefficient of skin surface reflection and the wavelength of the electromagnetic radiation, to which the skin surface part has been exposed, further illustrating the same curve A as shown in Fig. 1 and a curve C illustrating the response of a non-erythrodermic and extremely pigmented skin surface part of an individual,

Fig. 3 is a diagram illustrating the linear relationship between the logarithmic representation of a number of individuals' coefficients of skin surface reflection and the individuals' ability to stand exposure to ultraviolet radiation of a predetermined intensity and of a predetermined specific spectral composition,

Fig. 4 is an overall schematic and perspective view of an apparatus implemented in accordance with the teaching of the present invention,

Fig. 5A is an overall schematic and partly cut-away view of a photodetector constituting a component of the apparatus shown in Fig. 4,

Figs. 5B and 5C are elevational views of an optic guide and light diode and light detector supporting component of the photodetector shown in Fig. 5A.

Fig. 6 is a diagram illustrating the relation between measurements carried out on a total of 49 individuals, further illustrating the correspondence between the coefficient of reflection to red light and the coefficient of reflection to green light in logarithmic representation in dependency of the blood flow,

Fig. 7 is a diagram similar to the diagram shown in Fig. 6, from which diagram an individual's corrected coefficient of reflection to red light and the same individual's pigmentation may be determined from a set of measuring results and related to a specific state of redness,

Fig. 8 is a diagram similar to the diagrams of Figs. 6 and 7, illustrating the relation between the coefficient of reflection to red light and the coefficient of reflection to green light in logarithmic representation of individuals of different degrees of redness caused by ultraviolet radiation,

Fig. 9a is a diagram illustrating the relation between the correspondence between the corrected coefficient of reflection to red light in logarithmic representation of individuals and the UV dose in B-MED of the same individuals,

Fig. 9b is a diagram illustrating the relation between the correspondence between the pigment% and the UV-dose in B-MED of the same individuals,

Fig. 10 is a diagram illustrating the linear correspondence between clinically evaluated redness of individuals and the degree of redness of the same individuals,

Fig. 11 is a circuit diagram of the electronic circuitry of the apparatus shown in Fig. 4,

Fig. 12 A and B are circuit diagrams of a microprocessor block of the electronic circuitry shown in Fig. 11,

Fig. 13 is a circuit diagram of the electronic circuitry of the photodetector shown in Fig. 5,

Fig. 14 is a diagram illustrating the linear relation between laser-induced skin changes of individuals subjected to laser treatment and the degree of pigmentation of the same individuals,

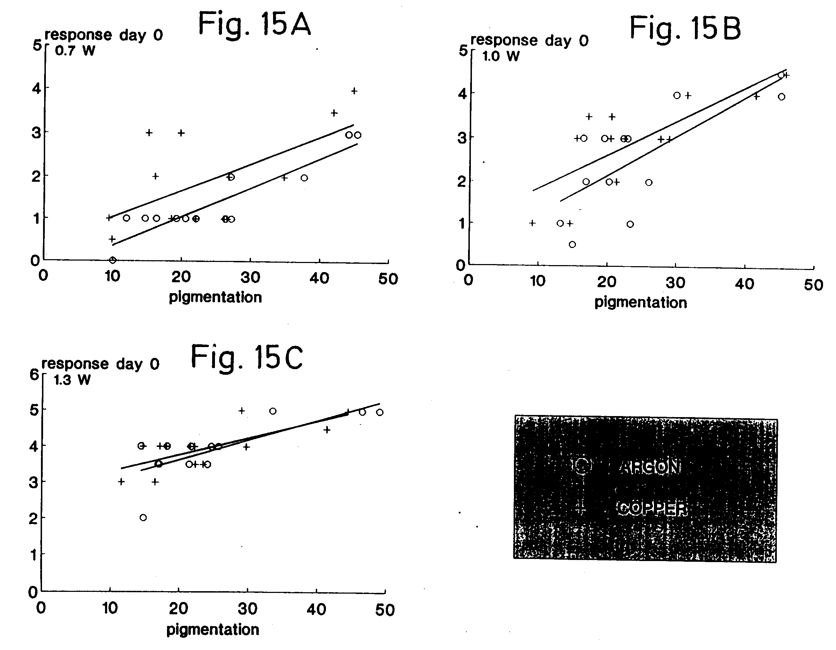

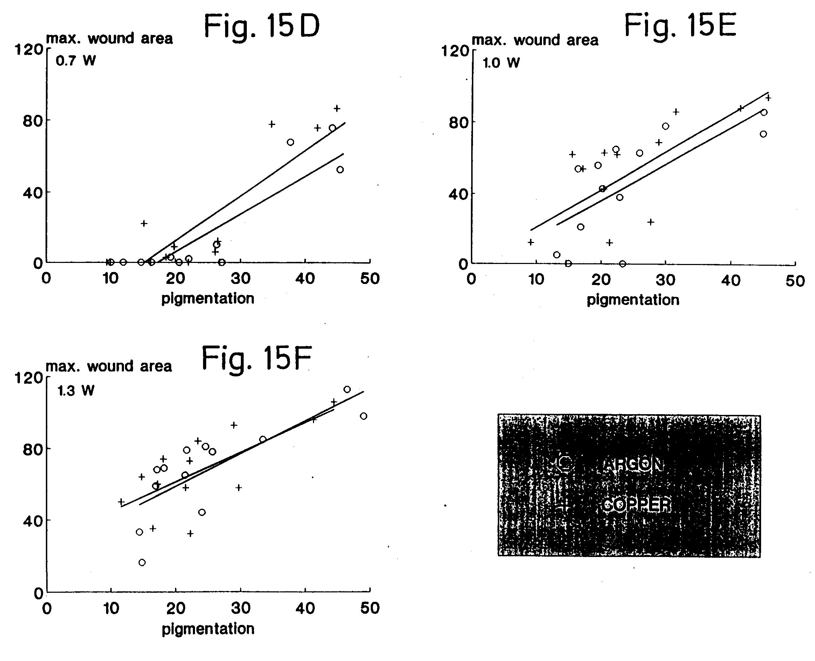

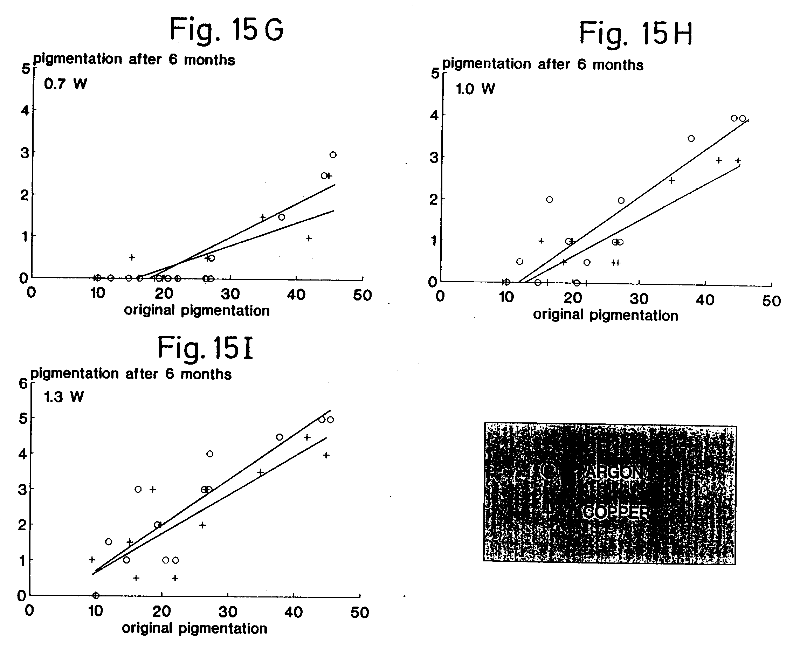

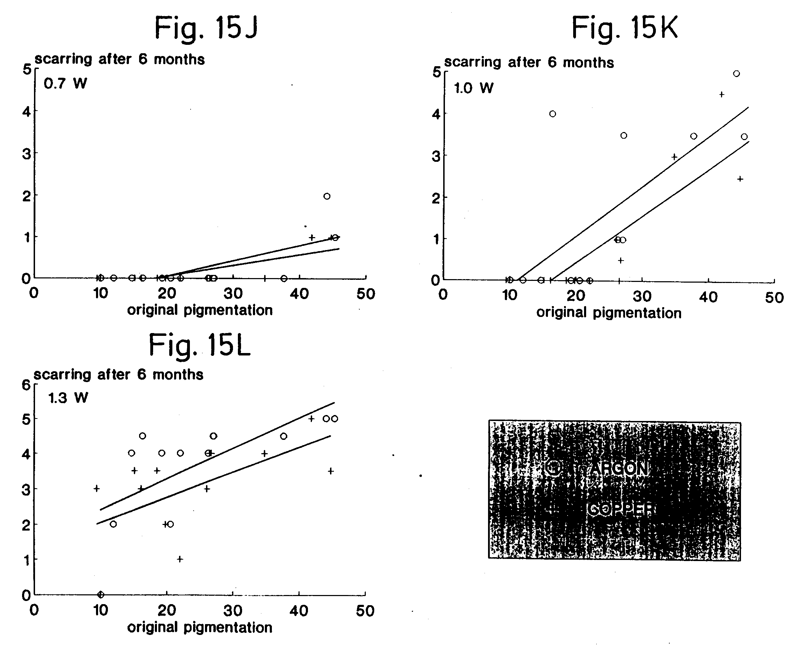

Fig. 15A-15L are diagrams further illustrating the correspondence between the laser-induced effects and the degree of pigmentation of the individuals exposed to laser treatment,

Fig. 16 a diagram illustrating the linear relationship between individuals' basic MEDs after UV treatment and the degree of pigmentation of the same individuals,

Fig. 17 is a diagram illustrating the difference between an experimentally found dose and a calculated dose to reach a certain degree of redness, and

Fig. 18 is a diagram illustrating the difference of an individual's sensitivity to UV treatment compared to a normal sensitivity.

According to the present invention, non-invasive measuring techniques for measuring pigmentation and redness of a skin surface part of an individual are provided. Basically, the present invention constitutes a refinement or improvement of the technique described in US Patent No. 4.288.598, to which US Patent reference is made. According to the non-invasive measuring technique according to the above US Patent and according to the present invention, a conversion from pigmentation to UV-sensitivity is established and an objective measurement of pigmentation and redness is provided, which objective measurement may be converted into UV-sensitivity of the individual in question. According to the teachings of the present invention, phototherapy and photochemotherapy during treatment is adjustable so as to optimize the treatment of an individual at all times, taking into due consideration the action spectra for phototherapy and photochemotherapy.

The basic teachings of the present invention are based on the below described measurements.

Spectrophotometric measurements of the reflection of skin surface parts of individuals were carried out within the wavelength area 260 nm - 800 nm. A laboratory system comprising a 150 W Xenon arc lamp (manufactured by Zeiss, FRG) was used irradiating the entrance slit of a single grating monochromator (manufactured by Jobin-Yvon H20, France, 200 nm focal length, f/3.5). An 8 nm band-pass filter was used for all measurements. From the exit slit of the monochromator the light beam was directed to the skin surface part of an individual via one branch of a fused silica bifurcated fiber optic cable (manufactured by Oriel, Stratford, CT, USA). Said one branch of the fiber optic cable was held perpendicular to the skin surface part at a distance of 5 mm by means of a spacer device. The other branch of the fiber optic cable was used for directing the radiation reflected from the skin surface part to a calibrated multiprobe (manufactured by EG & G 550 - 2) connected to a radiometer (manufactured by EG & G 550, Salem, MA, USA). The measurements were performed at every second nm from 260 nm to 800 nm using the reflection from a calcium carbonate plate as the 100% reflection reference at all wavelengths. On the basis of the measurements, spectra were recorded, and calculations based on the measurements were performed by a HP computer (manufactured by Hewlett Packard, Palo Alto, CA, USA).

A total of 22 individuals were used for recording reflection spectra, which 22 individuals had different skin complexion. Within the skin surface areas of the 22 individuals within which skin surface areas the measurements were performed, the MED (Minimum Erythema Dose for an individual person 24 h after exposure) was determined using the radiation from a Solar Simulator (manufactured by Solar Light Company, Philadelphia, PA, USA). Spots of a diameter of 1 cm were irradiated, and the redness of the skin surface parts was classified as one MED when minimal redness had clearly demarcation lines to the surrounding non-irradiated skin.

For all 22 individuals, spectra were recorded from closely spaced minimally pigmented and pigmented areas. A comparison of these spectra showed that the best discrimination between the curves was between 507 nm and 512 nm.

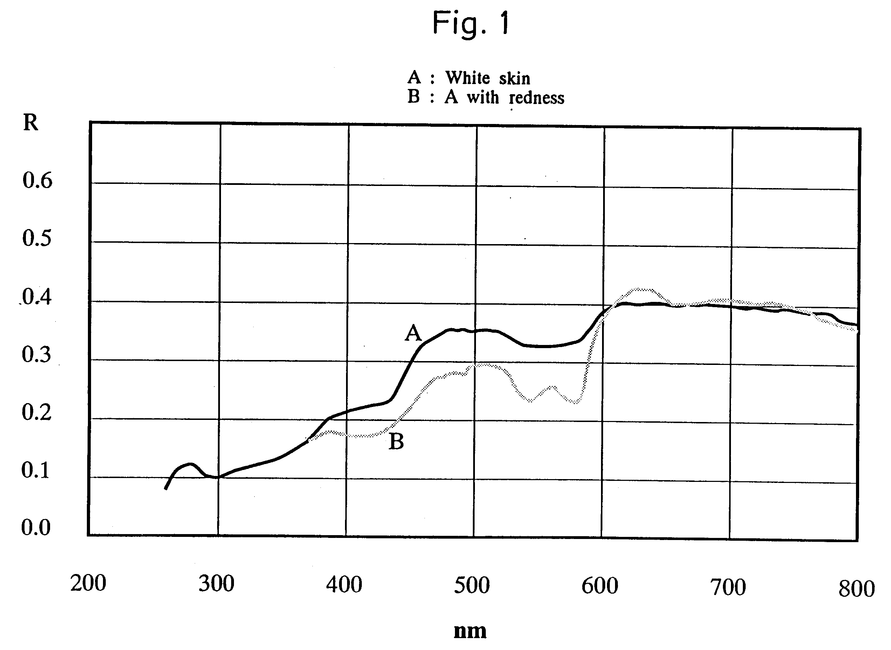

In Fig. 1, a diagram is shown illustrating the correspondence between the coefficient of skin surface reflection and the wavelength of the electromagnetic radiation, to which the skin surface part has been exposed and illustrating a curve A representing the response of an average non-sun-tanned and non-erythrodermic skin surface part of an individual and a curve B representing the response of an erythrodermic or sun-burned skin surface part of an individual. The maximum difference between the curves A and B was between 503 nm and 512 nm for all individuals.

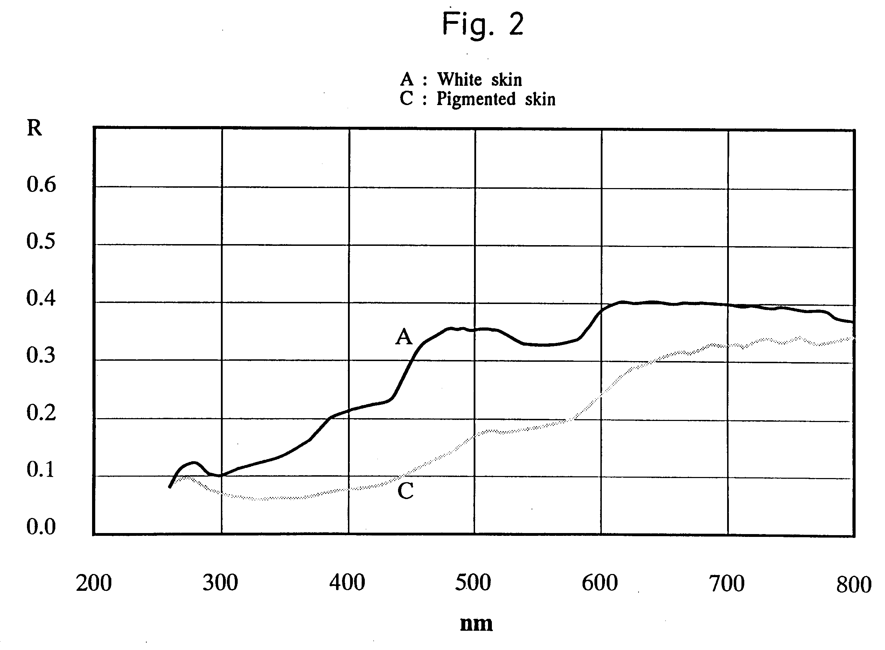

In Fig. 2, a diagram similar to the diagram of Fig. 1 is shown, comprising the curve A also shown in Fig. 1 and further a curve C representing the response of a non-erythrodermic and extremely pigmented skin surface part of an individual. The maximum difference between the curves A and C was between 503 and 512 nm.

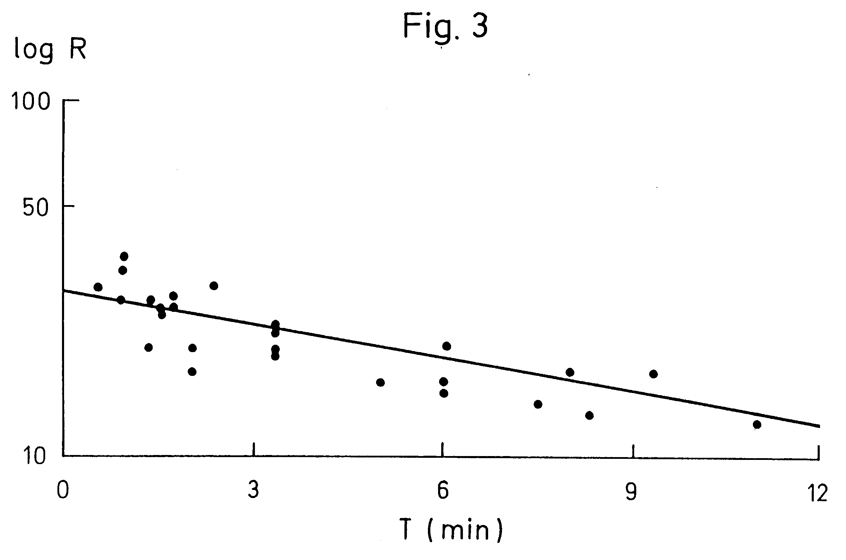

In Fig. 3 a diagram is shown, which diagram corresponds to the diagram shown in Fig. 1 in US Patent No. 4.882 598, and which diagram illustrates the linear relationship between the coefficient of reflection to light of a wavelength of 510 nm in logarithmic representation and the time to reach 1 MED using the Solar Simulator recorded for a total of 26 individuals.

On basis of the measuring results illustrated by the curves A, B, and C shown in Figs. 1 and 2, it is concluded that redness influences the measurements of pigmentation when wavelengths between abt. 380 nm to abt. 600 nm are used. Also the valleys corresponding to the absorption of hemoglobin (abt. 540 - 580 nm) are clearly observed. Reflection within the wavelength area 540 - 580 nm is consequently to be used for estimating redness of the skin and wavelengths above approx. 600 nm and below approx. 380 nm should be used for estimating pigmentation as measurements at these wavelengths are to no substantial extent affected by redness.

Consequently, light sources to be chosen for this purpose are e.g. light-emitting diodes with peak wavelengths at 550 nm (Green) and 660 nm (Red).

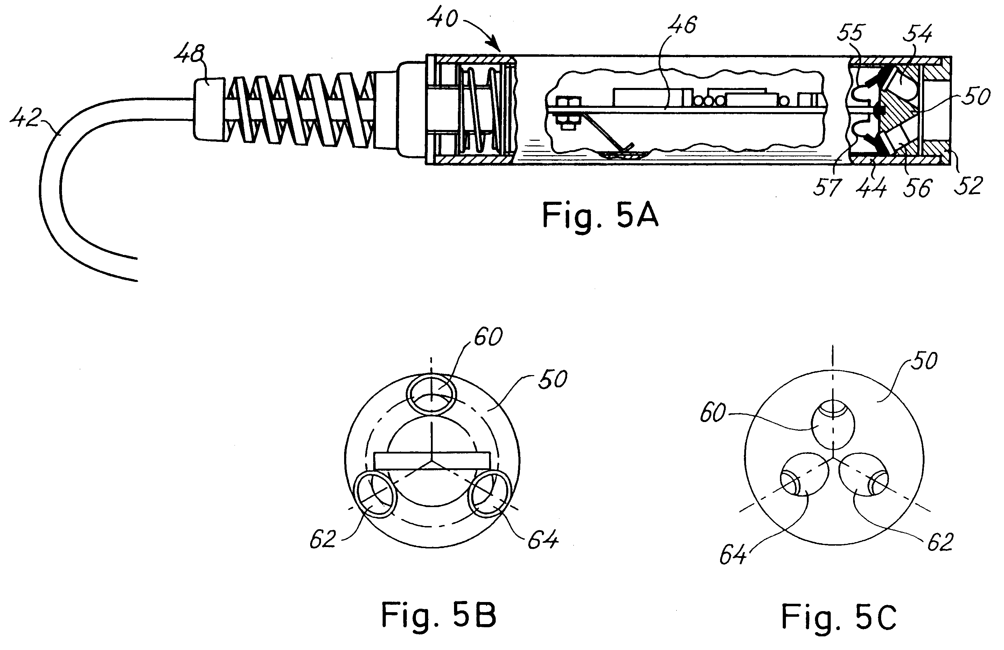

A unique measuring probe comprising two light-emitting diodes emitting light of the above wavelengths was designed, which measuring probe is shown in Fig. 5 and is to be discussed in greater detail below.

A further experiment involving 49 volunteers with different pigmentation was made, which experiment revealed relations between the skin surface reflection of red light and green light, which relations constitute the basic realization of the present invention.

In order to investigate in what way redness of the skin influences the estimation of pigmentation, the following test was conducted:

For the above-mentioned 49 individuals green reflection and red reflection were recorded on the antebrachium both on the volar side and on the dorsal side, provided the skin surface of the individual was not too hairy. A total of four situations were tested in one and the same spot ensuring a constant pigmentation. The four situations were: Zero flow, the arm in vertical position, the arm in horizontal position, and a situation in which reactive redness was generated after Zero flow. Zero flow was obtained by pressing all blood out of the arm using elastic bandage followed by obstruction of the blood flow by a cuff on the upper arm. In all cases, the individual was lying in a horizontal position and the skin changes were monitored until the changes had stabilized.

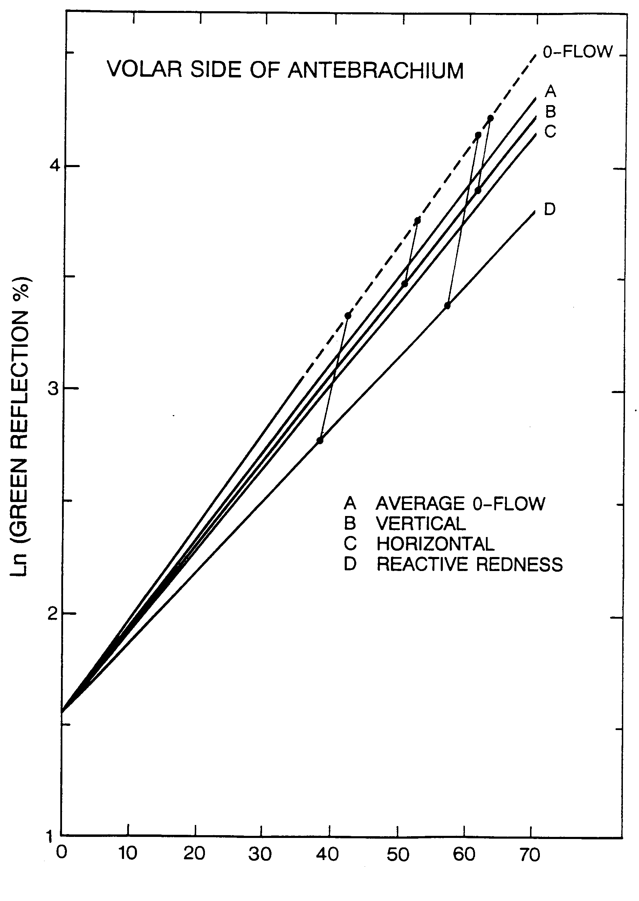

Fig. 6 is a diagram in which the coefficient of skin reflection to red light and the skin reflection to green light in logarithmic representation are listed for all 49 individuals. From the measuring results, it is first of all verified that the coherent sets of skin surface reflection to red light and skin surface reflection to green light in logarithmic representation of the four above listed situations are positioned along straight lines A, B, C, and D, respectively, having a common intercept at 1.54 at the ordinate axis. The slope of the curve A representing the average Zero flow was deduced from the diagram being 0.03977, and the slope of the straight line D representing the reactive redness situation was also deduced from the diagram being 0.03247. The straight lines B and C representing the vertical and the horizontal position of the arm of the individual, respectively, had slopes in between the slopes of the straight lines A and D. For one and the same individual, the pigmentation should be considered constant in all four situations, and the red reflection should consequently be identical in all four situations. This is, however, not the case. The test revealed that the measurements corresponding to the above described four situations, i.e. corresponding to the straight lines A, B, C, and D, performed on a single individual could be presented by a straight line which, however, was not a vertical line in the diagram shown in Fig. 6. Examples of the straight line corrections are shown in Fig. 6 which straight lines connect points on the Zero flow line and the corresponding points on the lines B, C, and D corresponding to the above described situations. On the basis of the straight line correction, any set of measuring results representing the skin surface reflection to red and the skin surface reflection to green in logarithmic representation may be, so to speak, normalized as far as redness is concerned by correcting the measuring results to a normalized or standard situation, e.g. a Zero flow situation.

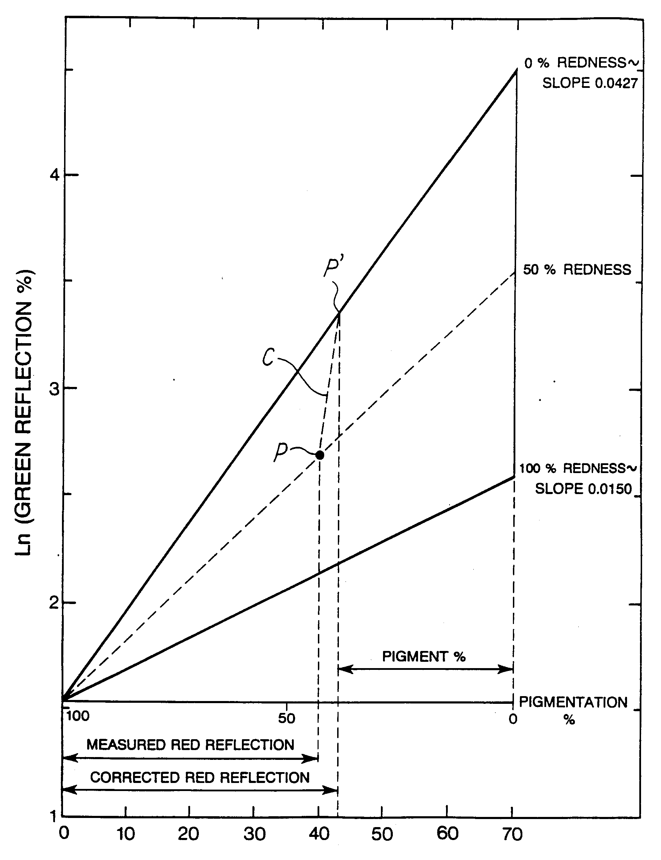

In Fig. 6, a straight dotted line O is also shown, representing the average Zero flow plus 2 SD (Standard Deviation) corresponding to a slope of 0.0427, including 95% of the Zero flow measurements. By converting any measuring results representing the skin surface reflection to red and the skin surface reflection to green in logarithmic representation to the straight, dotted line O, the best possible correction for redness of the skin for calculating the pigmentation may be performed, as will be evident from the discussion below with reference to Fig. 7. An extremely white individual exhibit a skin surface reflection to red of 70% which is consequently named 0% pigmentation, whereas 100% pigmentation corresponds to 0% skin surface reflection to red.

In Fig. 7, redness and pigmentation of a specific individual are calculated on the basis of skin surface reflection measurements to red and green light, the skin surface reflection to green light being presented in logarithmic representation. Thus, Fig. 7 is a diagram, the abscissa and ordinate axes of which are identical to the abscissa and ordinate axes of the diagram shown in Fig. 6. The straight line correction discussed above of the measuring results generated by measuring the coefficients of skin surface reflecction to red and green light of an individual and by converting the green light skin surface reflection coefficient into logarithmic representation is performed along a dotted line C, so to speak transforming the measuring results represented by the point P to a point P' of the line O, i.e. the line intercepting the ordinate at 1.54 and having a slope of 0.0427. The abscissa of the point P' represents the corrected red reflection coefficient and also the pigmentation degree represented in a percentage varying from 0% to 100% as indicated in Fig. 7. Consequently, a specific degree or state of redness for different individuals of different pigmentation is represented by a straight line of constant slope intercepting the ordinate at 1.54. Thus, the degree or state of redness of an individual is readily measurable on the basis of the diagram shown in Fig. 7, as a specific degree or state of redness corresponds to a specific straight line intercepting the ordinate at 1.54 and having a specific slope.

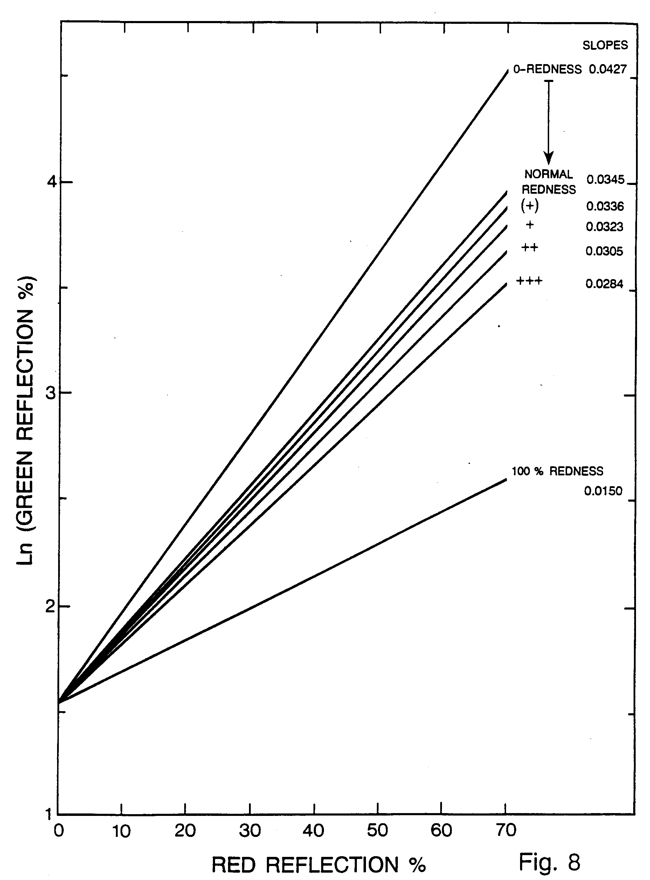

In Fig. 8, a diagram similar to the diagrams shown in Figs. 6 and 7 is shown, representing lines representing different degrees of redness caused by ultraviolet radiation exposed to individuals having different pigmentation. The lines intercept the ordinate axis at 1.54 like the straight lines shown in Figs. 6 and 7. The highest degree of redness corresponding to 100% redness is chosen to include individuals having naevus flammeus of very dark-bluish red colour and corresponds to a straight line of a slope of 0.015.

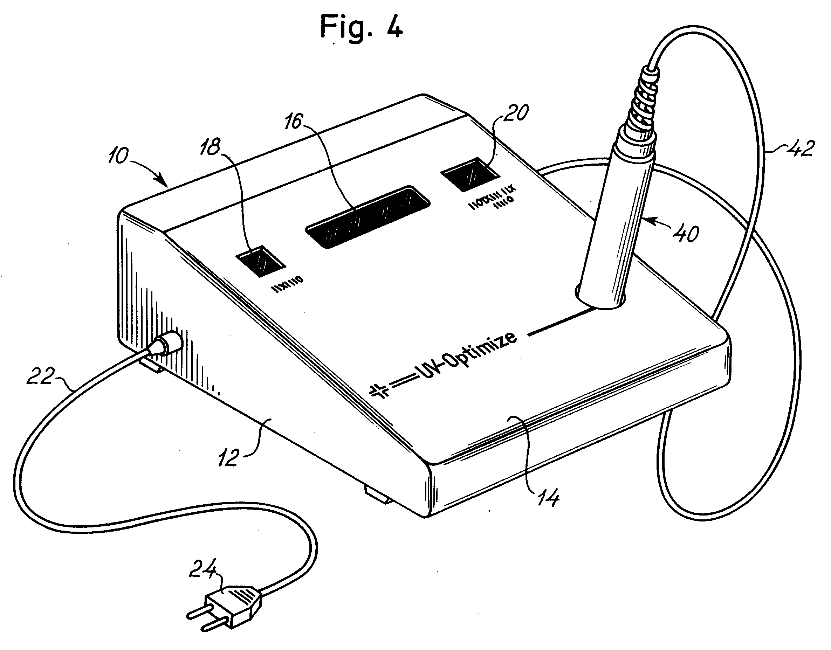

Table 1 below illustrates the correspondence between clinical redness as defined below, redness percentage and the read-out from an apparatus to be described below with reference to Fig. 4 and implemented in accordance with the teachings of the present invention.

Table 1

| Clinical Redness | Redness % | Display Reading |
|---|---|---|
| 0 | <29.6 | <30 |
| (+) | 32.7 | 33 |
| + | 37.4 | 37 |
| ++ | 43.9 | 44 |
| +++ | 51.6 | 52 |

Zero redness corresponds to the highest UV dose of Philips TL12 tubes, which dose may be given before redness appears.

(+) redness corresponds to faint, spotted redness without a clear demarcation to the surrounding, non-irritated skin surface areas.
+ redness corresponds to faint redness with a clear demarcation to the surrounding skin surface areas.
++ redness corresponds to clear redness with slight edema to be felt in the tissue.
+++ redness corresponds to heavy redness with edema to be felt or seen above the surrounding skin surface areas.

The measurements were made after having irradiated the 49 individuals on the buttocks with different doses of UV from Philips TL12 lamps. The doses used were in B-MED (Basic Minimal Erythema Dose being 312 $J/m^2$ at 296 nm. ((24 h erythema) Parrish). 24 h after irradiation, the redness degree was estimated by means of the above listed "+" system.

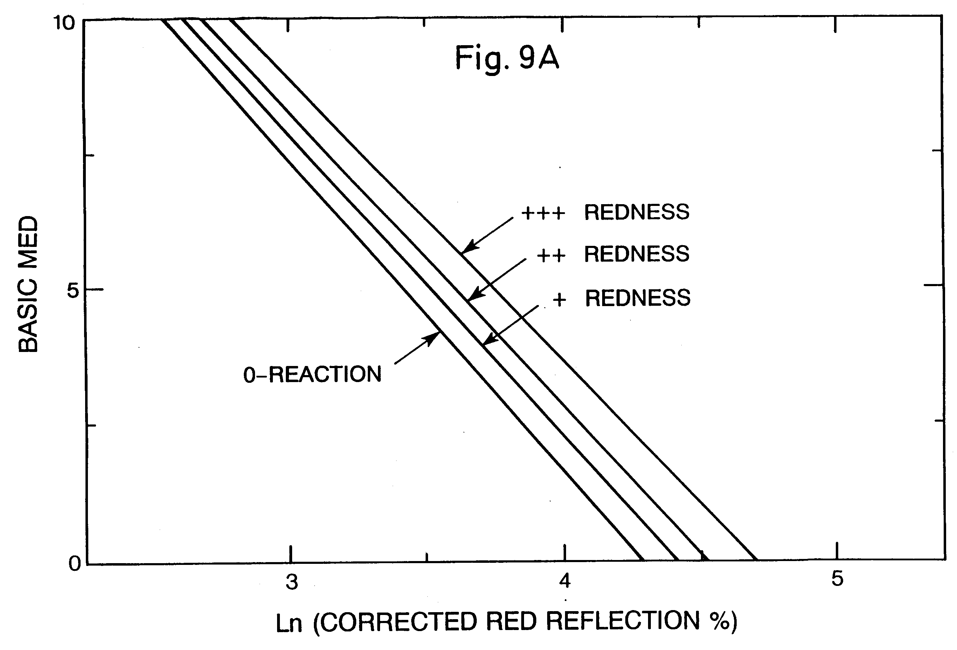

In Fig. 9a, a diagram is shown, illustrating the correspondence between the corrected coefficient of reflection to red and the UV dose in B-MED to reach different levels of erythema. Since all curves exhibit a common intercept at the ordinate axis, a common equation may be deduced which can be used to predict the treatment dose, taking into consideration the degree of pigmentation of the skin surface part in question and further what level of redness is to be produced 24 h after irradiation. The equation used is: Treatment time = 24.2 + (0.1709 x specific redness - 5.668) x ln KR (corrected coefficient of reflection to red) x number of seconds to reach a B-MED (the intensity of the light source). The sensitivity of a specific skin surface area of the individual's body relative to the tested part of the buttocks has to be taken into consideration when predicting the treatment dose.

The curves shown in Fig. 9a are almost parallel, and further calculations, according to which calculations the correspondence between the UV dose in B-MED is related to the degree of pigmentation, have proven that dose versus pigmentation may also be used to predict the treatment time. In this case, the additional dose to shift from UV reaction of 0 to +, from + to ++, and from ++ to +++ were identical and independent of pigmentation (0.69 B-MED). Under these circumstances, the equation is: Treatment time = (-0.206 + 0.689 x ord. redness (ord. means prescribed) + 0.0829 x pigmentation) x the number of seconds to reach a B-MED (the intensity of the light source).

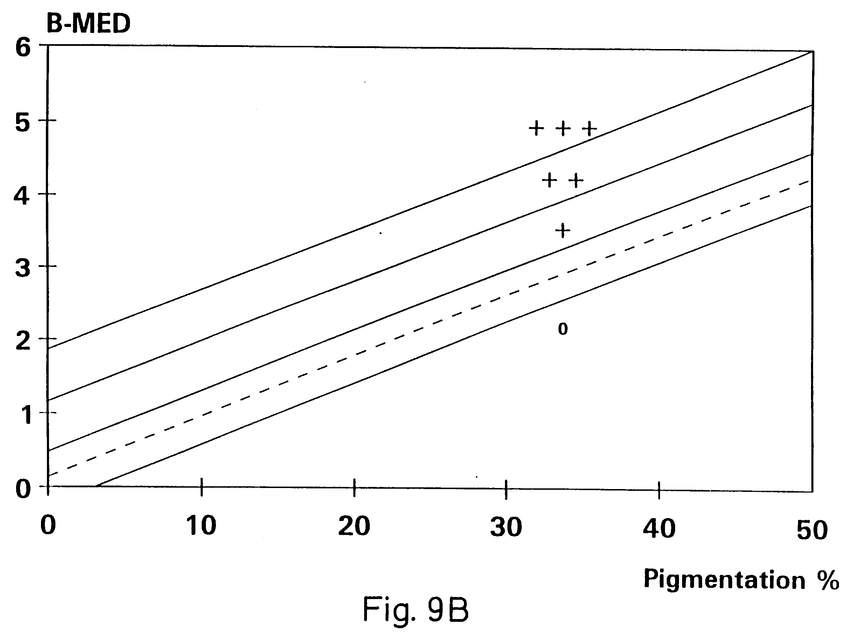

In Fig. 9b, a diagram is shown, illustrating the correspondence between pigmentation% and the UV-dose in B-MED to reach different levels of erythema. Since all curves are parallel, the curves shown in Fig. 9b are preferably used instead of the curves shown in Fig. 9a, thus substituting the curves shown in Fig. 9a in calculations similar to the above calculations.

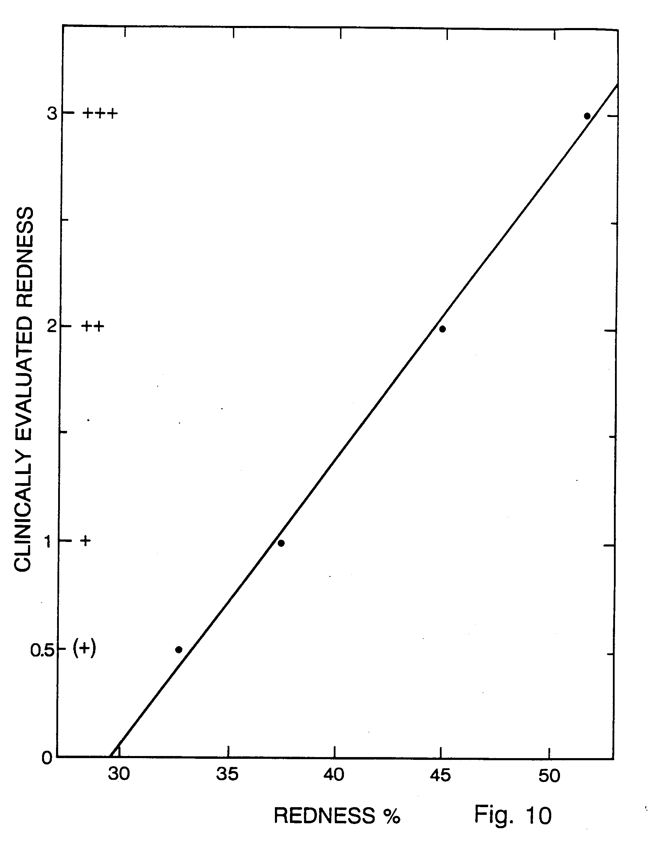

The redness "plus" system may be converted into specific figures or numbers as illustrated in Fig. 10, which illustrates the relation between estimated redness and the degree or state of redness in % measured in accordance with the present invention and represented by straight lines.

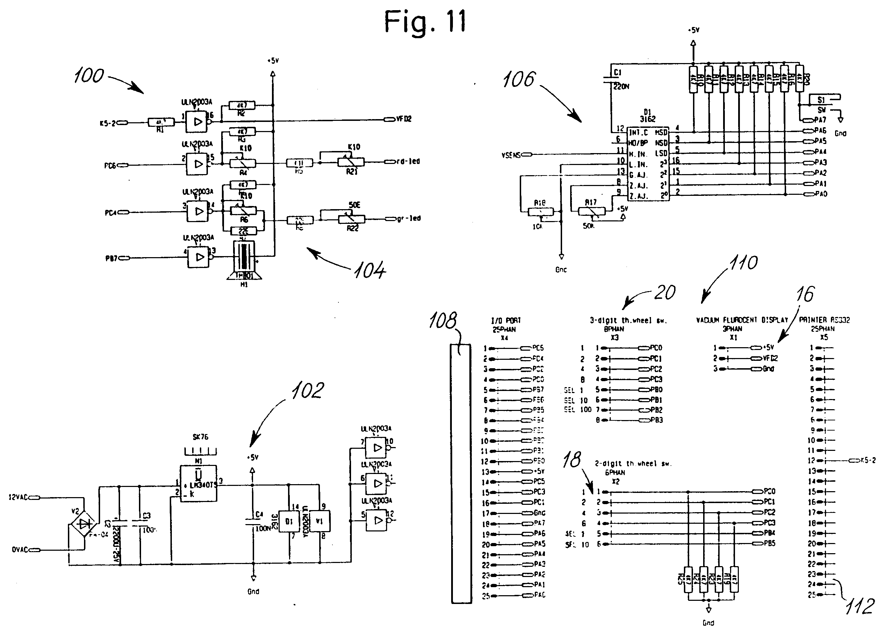

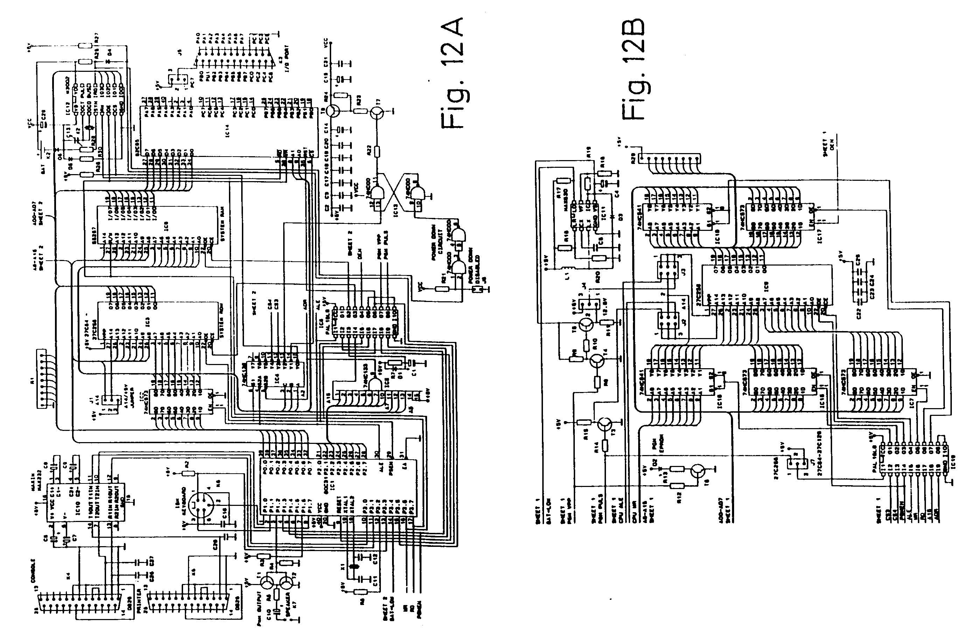

In Fig. 4 an overall perspective view of an apparatus according to the present invention is shown, which apparatus is designated the reference numeral 10 in its entirety. The apparatus 10 comprises a housing 12 defining a sloping top surface 14 in which a display 16 is arranged together with a 2-digit thumb wheel switch 18 and a 4-digit thumb wheel switch 20. The top surface 14 is further provided with a recess in which a photodetector 40 to be described in greater details below with reference to Fig. 5 is received in the idle mode of the apparatus. The photodetector 40 is connected to the electronic circuitry of the apparatus 10 through a multicore cable 42. The apparatus 10 is a mains supplied apparatus and is consequently provided with a mains cable 22 and a mains plug 24. The electronic circuitry of the apparatus 10 is to be described in greater details below with reference to Figs. 11, 12A, 12B, and Example 1.

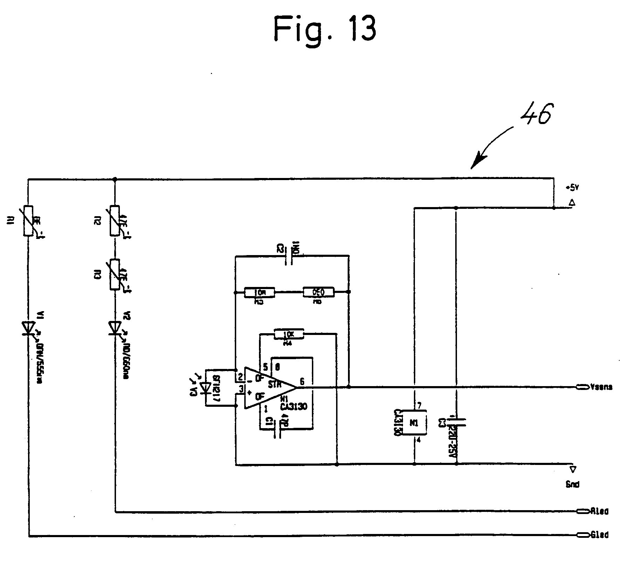

In Fig. 5A, a schematic and partly cut-away view of the photodetector 40 is shown. The photodetector 40 comprises a cylindrical housing 44 in which a printed circuit board 46 is received, which printed circuit board includes the electronic circuitry of the photodetector 40, which electronic circuitry is to be described in greater details below with reference to Fig. 13 and Example 1. The electronic circuitry of the printed circuit board 46 is connected to the multicore cable 42 which extends from the left-hand end of the cylindrical housing 44 through a coiled bushing 48. At the opposite, right-hand end of the cylindrical housing 44, a light guide and light-emitting diode supporting and light detector supporting component 50 is received, supported in a fixed position relative to the right-hand end of the photodetector 40 by means of an annular support component 52. In Fig. 5A, a light-emitting diode 54 and a light detector 56 are illustrated received within the component 50 and connected to the printed circuit board 46 through conductors 55 and 57, respectively.

In Figs. 5B and 5C, the component 50 is shown in greater details from the left-hand side and the right-hand side, respectively, relative to the position of the component 50 within the cylindrical housing 44 shown in Fig. 5A. From Figs. 5B and 5C, it is evident that a total of three apertures are provided extending through the component 50 for receiving two light-emitting diodes, one of which emits red light and one of which emits green light, and a light detector. The through-going apertures of the component 50 are designated the reference numerals 60, 62, and 64, respectively. The through-going apertures 60, 62, and 64 are geometrically spaced so as to minimize the risk that any false light may influence the light reflection measurement to be carried out by means of the photodetector 40 in accordance with the teaching of the present invention and also in accordance with the teaching described in the above-mentioned US Patent No. 4.882.598. The apertures 60, 62, and 64 are further arranged in a specific angular and sloping relation so as to obtain an optimum light transmission path from the light-emitting diodes to the light detector from a reflecting surface and further so as to eliminate any light scattering effects from light scattering surfaces, which light scattering might erroneously influence the measurement to be carried out by means of the photodetector 40 and the apparatus 10 according to the present invention, to which apparatus the photodetector 40 is connected.

In Fig. 11, an overall circuit diagram of the electronic circuitry of the apparatus 10 implemented in accordance with the teaching of the present invention is shown. The electronic circuitry is in its entirety designated the reference numeral 100. The electronic circuitry 100 basically comprises the following sections, a mains supply section 102, a photodetector input section 104, an A/D (Analog/Digital) converter section 106, a central microprocessor section or block 108, and an interface section 110 through which interface section, the sections 102, 104, 106, and 108 are connected to one another and further interfaced to the display 16, the 2-digit thumb wheel switch 18, the 4-digit thumb wheel 20, and a printer output port 112.

The components of the electronic circuitry 100 are listed in the below Example 1.

In Fig. 12, an overall circuit diagram of the microprocessor section or block 108 is shown, which microprocessor block is implemented by an electronic circuit of the type MCS 52, manufactured by the Danish company Circuit Design. The individual components of the microprocessor block or section 108 are listed in the below Example 1.

In Fig. 13, a circuit diagram of the electronic circuitry of the printed circuit board 46 of the photodetector 40 is shown. The individual components of the electronic circuitry 46 are listed in the below Example 1.

## Example 1

The electronic circuitry 100 of the apparatus 10 implemented in accordance with the teaching of the present invention was constructed from the components identified in Fig. 11.

The microprocessor block or section 108 of the electronic circuitry 100 was implemented by a circuit of the type MCS-52 supplied by the Danish company Circuit Design. The electronic components of the circuit diagram shown in Fig. 12 were:

| | |
|---|---|
| R1 | 4.7 kohm |
| R2-R4 | 10 kohm |
| R5 | 47 ohm |
| R6 | 10 kohm |
| R7 | 1 kohm |
| R8 | 10 kohm |
| R9-R11 | 1 kohm |
| R12 | 10 kohm |
| R13 | 220 ohm |
| R14 | 10 kohm |
| R15 | 1 kohm |
| R16 | 150 kohm |
| R17 | 10 kohm |
| R18 | 47 kohm |
| R19 | 470 kohm |
| R20 | 100 kohm |

| R21-R22 | 10 kohm |
|---|---|
| R23 | 47 ohm |
| R24 | 4.7 kohm |
| R25-R28 | 10 kohm |
| R29 | 4.7 kohm |
| R30 | 1 kohm |
| C1 | 100 uF |
| C2-C3 | 100 nF |
| C4 | 220 uF |
| C5 | 47 pF |
| C6-C9 | 22 uF |
| C10 | 100 uF |
| C11-C12 | 22 pF |
| C13 | 22 pF |
| C14-C15 | 100 uF |
| C16-C25 | 100 nF |
| C26-C28 | 470 pF |
| C29 | 1 uF |
| D1 | 1N4148 |
| D2 | red LED |
| D3 | IN4148 |
| D4-D6 | IN4148 |
| TI | BC547 |
| T2 | BC557 |
| T3-T4 | BC547 |
| T5 | BC557 |
| T6-T7 | BC547 |
| T8 | BC640 |
| XI | 7.3728 MHz |
| X2 | 32 kHz |
| LI | 150 uH |
| ICI | 80C31 |
| IC2 | 74HC573 |
| IC3 | 27C64(27C256) |
| IC4 | 74HC138 |
| IC5 | 74HC133 |
| IC6 | PAL16L8 |
| IC7 | 74HC573 |
| IC8 | 55257 |
| IC9 | 27C64(27C256) |
| IC10 | MAX232 |
| IC11 | MAX630 |
| IC12 | M3002 |
| IC13 | 74HC00 |
| IC14 | 82C55 |
| IC15 | 74HC573 |
| IC16 | 74HC541 |
| IC17 | 74HC573 |
| IC18 | 74HC541 |
| IC19 | PALI6L8 |

The photodetector 40 was constructed from the components identified in Fig. 13.

The apparatus according to the present invention fulfilled the below technical specifications:

9

| Light source | |
|---|---|
| LED green | 550 nm peak value |
| LED red | 660 nm peak value |
| **Power Requirements** | |
| Power | 220 V |
| **Dimensions** | |
| Weight | kg |
| Height, length and depth | 71x234x272 mm |
| **Environment** | |
| Ambient Temperature | 17 - 28°C |
| Humidity | 40 - 80% RH |
| **Max. variability between measurements under standardized conditions** | |
| On brown tile: | |
| Green LED | 0,2% |
| Red LED | 0,3% |
| On white standards: | |
| Green LED | 0,3% |
| Red LED | 0,6% |
| Max. interapparatus variability | +/- 2,5% on skin |
| Measuring time (from 1st to last beep) | -4,5 sec |

Example 2

The microprocessor of the microprocessor section or block 108 of the apparatus according to the present invention was operated by means of the programme listed in Table 2 or alternatively operated by means of the programme listed in Table 3. This programme is working in the same way as the programme listed in Table 2 but is giving a warning when the patient is read instead of recalculation of the dose. That allows the operator or the doctor to decide if any reduction in treatment time should be performed.

Provided the 2-digit thumb wheel is set to 15, the number of joules/sqcm will be calculated for PUVA-treatment (-300 + 2200 x ord. redness (ord. means prescribed) + 205 x pigment%). This dose should come from a Philips TL 09 lamp or a lamp with a similar spectral distribution. Likewise, it is possible to have the treatment dose in joule or m joules/sqcm for different lamp types when the thumb wheel to the left in Fig. 4 is set to 1-13. 14 is the code used when only pigment protection factor, skin redness, and skin pigmentation is to be shown on the display.

## Table 2:

```
10      REM 25.1.1992 PRG2.BAS
20      REM 100% refl=900 mV
30      REM FFE7H=65511=CONTROLREG.
40      REM FFE6H=65510= C-PORTE
50      REM FFE5H=65509= B-PORTE.
60      REM FFE4H=65508= A-PORTE.
70      BAUD 2400 :   REM BAUDRATE TIL DISPLAY
80      XBY(65511)=145 :   REM A INPUT,B OUTPUT,C-LOW INPUT,C-HI OTPUT
90      GOSUB 2010
100     GOSUB 2030
110     PRINT #"
120     JUMPER=XBY(65508).AND.128 :   IF JUMPER=0 THEN 2140
130     FOR P=0 TO 5
140     XBY(65509)=2**P
150     SW(P)=XBY(65510).AND.15
160     NEXT P
170     SW2=1000*SW(3)+100*SW(2)+10*SW(1)+SW(0) :   REM S/MED FOR KILDEN
180     SW1=10*SW(5)+SW(4)
185     ORD=SW1*.1 :   REM ORDINERET 24H ROD (1 DECIMAL)
190     IF SW2=0 THEN 1630
200     XBY(65510)=0 :   REM DIODES OFF
210     F=0 : SB=0
220     N=0
230     GOSUB 640
240     IF M(N)>100 THEN 130
250     IF SW1>30 THEN 300
260     GOSUB 2010
270     GOSUB 2030
280     PRINT #"
290     GOTO 340
300     GOSUB 2010
310     GOSUB 2050
320     PRINT #"
330     GOTO 130
340     N=N+1
350     IF N<3 THEN 230
360     XBY(65509)=128 :   REM PB7=LYDGIVER ON
370     FOR T=0 TO 10 :   NEXT T
380     XBY(65509)=0 :   REM PB7=LYDGIVER OFF
390     XBY(65510)=16 :   REM PC4 HI GREEN DIODE ON.
400     FOR T=0 TO 200 :  NEXT T
410     FOR N=0 TO 4
420     GOSUB 640
430     G(N)=M(N)
440     NEXT N
450     XBY(65509)=128
460     FOR T=0 TO 10 :  NEXT T
470     XBY(65509)=0
480     XBY(65510)=0 :   REM  DIODES OFF
490     FOR T=0 TO 200 :   NEXT T
500     FOR N=0 TO 4
510     GOSUB 640
520     S(N)=M(N)
530     NEXT N
540     XBY(65509)=128
550     FOR T=0 TO 10 :   NEXT T
```

```
560    XBY(65509)=0
570    XBY(65510)=64 :  REM PC6 HI RED MODE ON.
580     FOR T=0 TO 200 :  NEXT T
590     FOR N=0 TO 4
600      GOSUB 640
610    R(N)=M(N)
620     NEXT N
630     GOTO 830
640     DO
650    I=XBY(65508) : C=I.AND.112
660     UNTIL C=112 :  REM I.E.PA6,PA5 OG PA4 HI.
670     DO
680    I=XBY(65508) : C=I.AND.112
690     UNTIL C=48 :  REM  I.E. PA6 LO I.E. HUNDREDS SELECTED.
700    H=I.AND.15
710     DO
720    I=XBY(65508) : C=I.AND.112
730     UNTIL C=96 :  REM I.E. PA4 LO I.E. UNITS SELECTED.
740    U=I.AND.15
750     DO
760    I=XBY(65508) : C=I.AND.112
770     UNTIL C=80 :  REM I.E. PA5 L I.E. TENS SELECTED.
780    T=I.AND.15
790    FT=1
800     IF H=10 THEN H=0 : FT=-1
810    M(N)=FT*(100*H+10*T+U)
820     RETURN
830    XBY(65509)=128 : XBY(65510)=0
840     FOR T=0 TO 100 :  NEXT T
850    XBY(65509)=0
860    GMIN=1000 : SMIN=1000 : RMIN=1000
870    GMAX=0 : SMAX=0 : RMAX=0
880    GS=0 : SS=0 : RS=0
890     FOR N=0 TO 4
900    GS=GS+G(N) : SS=SS+S(N) : RS=RS+R(N)
910     IF G(N)<GMIN THEN GMIN=G(N)
920     IF G(N)>GMAX THEN GMAX=G(N)
930     IF S(N)<SMIN THEN SMIN=S(N)
940     IF S(N)>SMAX THEN SMAX=S(N)
950     IF R(N)<RMIN THEN RMIN=R(N)
960     IF R(N)>RMAX THEN RMAX=R(N)
970     NEXT N
980     IF GMAX-GMIN>40 THEN F=1
990     IF SMAX-SMIN>10 THEN F=1
1000    IF RMAX-RMIN>40 THEN F=1
1010   RG=.1*INT((GS-SS)/4.5)
1020   RR=.1*INT((RS-SS)/4.5)
1030    IF RG<5 THEN F=1
1050    IF F=1 THEN 1180
1100   GI=LOG(RG)-1.54 :  REM ln(RG)-intercept
1110   SL=GI/RR :  REM slope
1120   KR=RR+17.5*(1-SL/.0427) :  REM KORR. RED REFL.
1130   KGI=.0427*KR :  REM KORR GR REFL
1150    IF KR=0 THEN F=1
1160    IF KR<0 THEN F=1
1170    IF F=0 THEN 1350
```

12

```
1180    GOSUB 2010
1190    GOSUB 2070
1200    GOSUB 2090
1210    FOR T=0 TO 600 :  NEXT T
1220    GOTO 90
1350  PMT=100*(70-KR)/70 :  IF PMT<0 THEN PMT=0
1360  REDN=100*(.0427-SL)/.0277 :  IF REDN<0 THEN REDN=0
1365   IF PMT>60 THEN SB=1
1370  PMT=INT(PMT)
1380  REDN=INT(REDN)
1390   PRINT #CHR(27),"I",CHR(17),CHR(22),
1400   PRINT #" PIGMENTATION ",PMT,"%"
1405   IF SB=1 THEN  PRINT #" REDNESS UNRELIABLE " :  GOTO 1420
1410   PRINT #" REDNESS      ",REDN,"%"
1420   FOR T=0 TO 1500 :  NEXT T
1430  PP=24.2-5.4971*LOG(KR) :  REM PIGM.BESL.FAKTOR
1440  PP=.1*INT(10*PP)
1450   PRINT #CHR(27),"I",CHR(17),CHR(22),
1460   PRINT #" PIGMENT PROTECTION"
1465   IF PP>.5 THEN 1470
1467   PRINT #"      UNRELIABLE     "
1468   FOR T=0 TO 1500 :  NEXT T
1469   GOTO 90
1470   PRINT #" FACTOR ",PP
1480   FOR T=0 TO 1500 :  NEXT
1485  ARD=16.97-491.5*SL :   REM  ACTUELL REDNESS
1487   IF PMT>60 THEN 1500
1490   IF REDN>29.6 THEN 1510
1500  TT=.5*(24.2+(.1709*ORD-5.668)*LOG(KR))*SW2 :  GOTO 1520
1510  TT=.1709*(ORD-ARD)*LOG(KR)*SW2
1520   PRINT #CHR(27),"I",CHR(17),CHR(22),
1530  TT=INT(TT) :   IF TT<0 THEN TT=0
1540  H=INT(TT/3600)
1550  M=INT((TT-3600*H)/60)
1560  S=TT-M*60-H*3600
1570   PRINT #"   TREATMENT TIME "
1580   IF H=0 THEN 1600
1590   PRINT #"  ",H,"HOUR",M,"MIN" :  GOTO 1622
1600   IF M=0 THEN 1620
1610   PRINT #"  ",M,"MIN ",S,"SEC   " :  GOTO 1622
1620   PRINT #"     ",S,"SEC      " :  GOTO 1622
1622   FOR T=0 TO 1500 :  NEXT T
1624   GOTO 120
1630   PRINT #CHR(27),"I",CHR(17),CHR(22),
1640   PRINT #"*CALIBRATE*   PLACE"
1650   PRINT #"DETECTOR ON STANDARD"
1660  XBY(65509)=1
1670  SW=XBY(65510).AND.15 :  IF SW>0 THEN 10
1680  XBY(65509)=2
1690  SW=XBY(65510).AND.15 :  IF SW>0 THEN 10
1700  XBY(65509)=4
1710  SW=XBY(65510).AND.15 :  IF SW>0 THEN 10
1720  XBY(65509)=8
1730  SW=XBY(65510).AND.15 :  IF SW>0 THEN 10
1740   FOR T=0 TO 1000 :  NEXT T
1750  XBY(65510)=32
```

13

```
1760    FOR T=0 TO 200 :   NEXT T
1770    GOSUB 640
1780   S=M(N)
1790    IF S>40 THEN 1630
1800   XBY(65510)=48
1810    FOR T=0 TO 200 :   NEXT T
1820    GOSUB 640
1830   G=M(N)
1840   GP=.1*INT((G-S)/.9)
1850   XBY(65509)=128
1860    FOR T=0 TO 5 :   NEXT T
1870   XBY(65509)=0
1880    PRINT #CHR(27),"I",CHR(17),CHR(22),
1890    PRINT #GP,"%", TAB (9),"GREEN       "
1900   XBY(65510)=96
1910    FOR T=0 TO 200 :   NEXT T
1920    GOSUB 640
1930   R=M(N)
1940   XBY(65510)=32
1950   RP=.1*INT((R-S)/.9)
1960   XBY(65509)=128
1970    FOR T=0 TO 15 :   NEXT T
1980   XBY(65509)=0
1990    PRINT #RP,"%", TAB (9),"RED        "
2000    GOTO 1660
2010    PRINT #CHR(27),"I",CHR(17),CHR(22),
2020    RETURN
2030    PRINT #"    *** READY ***    "
2040    RETURN
2050    PRINT #" CHECK DOSE SETTING"
2060    RETURN
2070    PRINT #"ERROR   ERROR   ERROR"
2080    RETURN
2090    PRINT #" REPEAT MEASUREMENT"
2100    RETURN
2110    REM ***
2120    REM ***
2130    REM ***
2140    REM TESTPG FOR HUDREFLEKTANSMETER.TEST.BAS
2150    REM *** INITIALISERING
2160    REM 100% refl=900 mV
2170    REM FFE7H=65511=CONTROLREG.
2180    REM FFE6H=65510= C-PORTE
2190    REM FFE5H=65509= B-PORTE.
2200    REM FFE4H=65508= A-PORTE.
2210    BAUD 2400 :   REM BAUDRATE TIL DISPLAY
2220   XBY(65511)=145 :   REM A INPUT,B OUTPUT,C-LOW INPUT,C-HI OUTPUT
2230    PRINT #CHR(27),"I",CHR(17),CHR(22),
2240    REM *** INIT SLUT
2250    PRINT #"TESTER LYDGIVER"
2260    FOR N=0 TO 1
2270   XBY(65509)=128
2280    FOR T=0 TO 50 :   NEXT T
2290   XBY(65509)=0
2300    FOR T=0 TO 50 :   NEXT T
2310    NEXT N
```

```
2320   PRINT #" TESTER OMSKIFTERE"
2330   DIM SW(6)
2340   FOR P=0 TO 5
2350   XBY(65509)=2**P
2360   SW(P)=XBY(65510).AND.15
2370   NEXT P
2380   FOR P=5 TO 4 STEP -1
2390   PRINT #SW(P),
2400   NEXT P
2410   PRINT #"   ",
2420   FOR P=3 TO 0 STEP -1
2430   PRINT #SW(P),
2440   NEXT P
2450   PRINT #
2460   SW2=1000*SW(3)+100*SW(2)+10*SW(1)+SW(0)
2470   SW1=10*SW(5)+SW(4)
2480   IF SW2=0 THEN 2500
2490   GOTO 2340
2500   PRINT #CHR(27),"I",CHR(17),CHR(22),
2510   PRINT #"  A/D-CONV.UDG. mV"
2520   GOSUB 2750
2530   PRINT #"   ",M, TAB (10),"              "
2540   PRINT #""
2550   XBY(65509)=1 : SW(0)=XBY(65510).AND.15
2560   IF SW(0)=1 THEN 2600
2570   IF SW(0)=2 THEN 2670
2580   GOTO 2520
2590   GOTO 2520
2600   XBY(65510)=16 :   REM PC4 HI GREEN DIODE ON.
2610   FOR T=0 TO 200 :   NEXT T
2620   GOSUB 2750
2630   XBY(65510)=0 :   REM  DIODES OFF
2640   PRINT #"   ",M, TAB (10),"GREEN      "
2650   PRINT #""
2660   GOTO 2550
2670   XBY(65510)=64 :   REM PC6 HI RED DIODE ON
2680   FOR T=0 TO 200 :   NEXT T
2690   GOSUB 2750
2700   XBY(65510)=0 :   REM    DIODES OFF
2710   PRINT #"   ",M, TAB (10),"RED        "
2720   PRINT #""
2730   GOTO 2550
2740   REM ***AFLAESNING AD A/D-CONVERTER
2750   DO
2760   I=XBY(65508) : C=I.AND.112
2770   UNTIL C=112 :   REM I.E.PA6,PA5 OG PA4 HI.
2780   DO
2790   I=XBY(65508) : C=I.AND.112
2800   UNTIL C=48 :   REM  I.E. PA6 LO I.E. HUNDREDS SELECTED.
2810   H=I.AND.15
2820   DO
2830   I=XBY(65508) : C=I.AND.112
2840   UNTIL C=96 :   REM I.E. PA4 LO I.E. UNITS SELECTED.
2850   U=I.AND.15
2860   DO
2870   I=XBY(65508) : C=I.AND.112
```

```
2880    UNTIL C=80 :  REM I.E. FA5 L I.E. TENS SELECTED.
2890    T=I.AND.15
2900    FT=1
2910     IF H=10 THEN H=0 : FT=-1
2920    M=FT*(100*H+10*T+U)
2930     RETURN
2940     REM *** AFLAESNING SLUT
```

## Table 3:

```
10      REM 18.1.1993 PRG2.BAS MOD TIL SPEC3.BAS
20      REM 100% refl=900 mV
30      REM FFE7H=65511=CONTROLREG.
40      REM FFE6H=65510= C-PORTE
50      REM FFE5H=65509= B-PORTE.
60      REM FFE4H=65508= A-PORTE.
70      BAUD 2400 :   REM BAUDRATE TIL DISPLAY
80      XBY(65511)=145 :   REM A INPUT,B OUTPUT,C-LOW INPUT,C-HI OUTPUT
90       GOSUB 2010
100      GOSUB 2030
110      PRINT #"              "
120     JUMPER=XBY(65508).AND.128 :   IF JUMPER=0 THEN 2140
130      FOR P=0 TO 5
140     XBY(65509)=2**P
150     SW(P)=XBY(65510).AND.15
160      NEXT P
170     SW2=1000*SW(3)+100*SW(2)+10*SW(1)+SW(0) :   REM S/MED FOR KILDEN
180     SW1=10*SW(5)+SW(4)
185     ORD=SW1*.1 :   REM ORDINERET 24H ROD (1 DECIMAL)
190      IF SW2=0 THEN 1630
200     XBY(65510)=0 :   REM DIODES OFF
210     F=0 : SB=0
220     N=0
230      GOSUB 640
240      IF M(N)>100 THEN 130
250      IF SW1>40 THEN 300
260      GOSUB 2010
270      GOSUB 2030
280      PRINT #"              "
290      GOTO 340
300      GOSUB 2010
310      GOSUB 2050
320      PRINT #"              "
330      GOTO 130
340     N=N+1
350      IF N<3 THEN 230
360     XBY(65509)=128 :   REM PB7=LYDGIVER ON
370      FOR T=0 TO 10 :   NEXT T
380     XBY(65509)=0 :   REM PB7=LYDGIVER OFF
390     XBY(65510)=16 :   REM PC4 HI GREEN DIODE ON.
400      FOR T=0 TO 200 :   NEXT T
410      FOR N=0 TO 4
420      GOSUB 640
430     G(N)=M(N)
440      NEXT N
450     XBY(65509)=128
460      FOR T=0 TO 10 :   NEXT T
470     XBY(65509)=0
480     XBY(65510)=0 :   REM  DIODES OFF
490      FOR T=0 TO 200 :   NEXT T
500      FOR N=0 TO 4
510      GOSUB 640
520     S(N)=M(N)
530      NEXT N
540     XBY(65509)=128
550      FOR T=0 TO 10 :   NEXT T
```

```
560    XBY(65509)=0
570    XBY(65510)=64 :   REM PC6 HI RED DIODE ON.
580     FOR T=0 TO 200 :   NEXT T
590     FOR N=0 TO 4
600     GOSUB 640
610    R(N)=M(N)
620     NEXT N
630     GOTO 830
640     DO
650    I=XBY(65508) : C=I.AND.112
660     UNTIL C=112 :   REM I.E.PA6,PA5 OG PA4 HI.
670     DO
680    I=XBY(65508) : C=I.AND.112
690     UNTIL C=48 :   REM  I.E. PA6 LO I.E. HUNDREDS SELECTED.
700    H=I.AND.15
710     DO
720    I=XBY(65508) : C=I.AND.112
730     UNTIL C=96 :   REM I.E. PA4 LO I.E. UNITS SELECTED.
740    U=I.AND.15
750     DO
760    I=XBY(65508) : C=I.AND.112
770     UNTIL C=80 :   REM I.E. PA5 L I.E. TENS SELECTED.
780    T=I.AND.15
790    FT=1
800     IF H=10 THEN H=0 : FT=-1
810    M(N)=FT*(100*H+10*T+U)
820     RETURN
830    XBY(65509)=128 : XBY(65510)=0
840     FOR T=0 TO 100 :   NEXT T
850    XBY(65509)=0
860    GMIN=1000 : SMIN=1000 : RMIN=1000
870    GMAX=0 : SMAX=0 : RMAX=0
880    GS=0 : SS=0 : RS=0
890     FOR N=0 TO 4
900    GS=GS+G(N) :  SS=SS+S(N) :  RS=RS+R(N)
910     IF G(N)<GMIN THEN GMIN=G(N)
920     IF G(N)>GMAX THEN GMAX=G(N)
930     IF S(N)<SMIN THEN SMIN=S(N)
940     IF S(N)>SMAX THEN SMAX=S(N)
950     IF R(N)<RMIN THEN RMIN=R(N)
960     IF R(N)>RMAX THEN RMAX=R(N)
970     NEXT N
980     IF GMAX-GMIN>40 THEN F=1
990     IF SMAX-SMIN>10 THEN F=1
1000     IF RMAX-RMIN>40 THEN F=1
1010   RG=.1*INT((GS-SS)/4.5)
1020   RR=.1*INT((RS-SS)/4.5)
1030     IF RG<5 THEN F=1
1050     IF F=1 THEN 1180
1100   GI=LOG(RG)-1.54 :   REM ln(RG)-intercept
1110   SL=GI/RR :   REM slope
1120   KR=RR+17.5*(1-SL/.0427) :   REM KORR. RED REFL.
1130   KGI=.0427*KR :   REM KORR GR. REFL
1150     IF KR=0 THEN F=1
1160     IF KR<0 THEN F=1
1170     IF F=0 THEN 1350
```

18

```
1180    GOSUB 2010
1190    GOSUB 2070
1200    GOSUB 2090
1210    FOR T=0 TO 600 :   NEXT T
1220    GOTO 90
1350  PMT=100*(70-KR)/70 :   IF PMT<0 THEN PMT=0
1360  REDN=100*(.0427-SL)/.0277 :   IF REDN<0 THEN REDN=0
1365    IF PMT>60 THEN SB=1
1370  PMT=INT(PMT)
1380  REDN=INT(REDN)
1390    PRINT #CHR(27),"I",CHR(17),CHR(22),
1400    PRINT #" PIGMENTATION ",PMT,"%"
1405    IF SB=1 THEN   PRINT #" REDNESS UNRELIABLE " :   GOTO 1420
1410    PRINT #" REDNESS        ",REDN,"%"
1420    FOR T=0 TO 2000 :  NEXT T
1430  PP=.483+.0829*PMT :   REM PIGM.BESK.FAKTOR
1440  PP=.1*INT(10*PP)
1450    PRINT #CHR(27),"I",CHR(17),CHR(22),
1460    PRINT #" PIGMENT PROTECTION"
1465    IF PP>.483 THEN 1470 :   REM IF PMT=0
1467    PRINT #"      UNRELIABLE     "
1468    FOR T=0 TO 2000 :   NEXT T
1469    GOTO 90
1470 ·  PRINT #" FACTOR ",PP
1480    FOR T=0 TO 2000 :   NEXT
1485  ARD=16.97-491.5*SL :   REM  ACTUELL REDNESS
1486    IF PMT<13 THEN PMT=13 :   REM TAALES ALTID
1488    IF SW2>0.AND.SW2<16 THEN   GOTO 3000
1490    IF PMT>60 THEN 1500
1491    REM  IF REDN>29.6 THEN 1510  erstattet af advarsel
1492    IF REDN<29.6 THEN 1500
1493    PRINT #CHR(27),"I",CHR(17),CHR(22),
1494    FOR T=O TO 4
1495    PRINT #" CHECK SKIN REDNESS "
1496    FOR P=0 TO 200 :   NEXT P
1497    PRINT #"              "
1498    FOR P=0 TO 50 :   NEXT P
1499    NEXT T
1500  TT=(-0.9+.689*ORD+.0829*PMT)*SW2
1502    IF SW15=1 THEN TT=-2300+2200*ORD+205*PMT : SW15=0
1503    REM -2000 PGA REGIONSFORSKELLE.
1506    GOTO 1520
1510    REM  TT=.689*(ORD-ARD)*SW2
1520    PRINT #CHR(27),"I",CHR(17),CHR(22),
1530  TT=INT(TT) :   IF TT<0 THEN TT=0
1531    IF LSW=0 THEN 1540
1532    PRINT #"    TREATMENT DOSE "
1533    IF TT<1000 THEN   PRINT #"    ",TT,"MJ/SQCM" :   GOTO 1537
1534  TT=INT(TT/100) : UVD=TT/10
1536    PRINT #"    ",UVD,"J/SQCM"
1537    FOR T=0 TO 2000 :   NEXT T
1538  LSW=0
1539    GOTO 120
1540  H=INT(TT/3600)
1550  M=INT((TT-3600*H)/60)
1560  S=TT-M*60-H*3600
```

```
1570    PRINT #"   TREATMENT TIME "
1580    IF H=0 THEN 1600
1590    PRINT #"   ",H,"HOUR",M,"MIN" :   GOTO 1622
1600    IF M=0 THEN 1620
1610    PRINT #"   ",M,"MIN ",S,"SEC   " :   GOTO 1622
1620    PRINT #"      ",S,"SEC     " :   GOTO 1622
1622    FOR T=0 TO 2000 :   NEXT T
1624    GOTO 120
1630    PRINT #CHR(27),"I",CHR(17),CHR(22),
1640    PRINT #"*CALIBRATE*   PLACE"
1650    PRINT #"DETECTOR ON STANDARD"
1660    XBY(65509)=1
1670    SW=XBY(65510).AND.15 :   IF SW>0 THEN 10
1680    XBY(65509)=2
1690    SW=XBY(65510).AND.15 :   IF SW>0 THEN 10
1700    XBY(65509)=4    .
1710    SW=XBY(65510).AND.15 :   IF SW>0 THEN 10
1720    XBY(65509)=8
1730    SW=XBY(65510).AND.15 :   IF SW>0 THEN 10
1740    FOR T=0 TO 1000 :   NEXT T
1750    XBY(65510)=32
1760    FOR T=0 TO 200 :   NEXT T
1770    GOSUB 640
1780    S=M(N)
1790    IF S>40 THEN 1630
1800    XBY(65510)=48
1810    FOR T=0 TO 200 :   NEXT T
1820    GOSUB 640
1830    G=M(N)
1840    GP=.1*INT((G-S)/.9)
1850    XBY(65509)=128
1860    FOR T=0 TO 5 :   NEXT T
1870    XBY(65509)=0
1880    PRINT #CHR(27),"I",CHR(17),CHR(22),
1890    PRINT #GP,"%", TAB (9),"GREEN       "
1900    XBY(65510)=96
1910    FOR T=0 TO 200 :   NEXT T
1920    GOSUB 640
1930    R=M(N)
1940    XBY(65510)=32
1950    RP=.1*INT((R-S)/.9)
1960    XBY(65509)=128
1970    FOR T=0 TO 15 :   NEXT T
1980    XBY(65509)=0
1990    PRINT #RP,"%", TAB (9),"RED         "
2000    GOTO 1660
2010    PRINT #CHR(27),"I",CHR(17),CHR(22),
2020    RETURN
2030    PRINT #"   *** READY ***    "
2040    RETURN
2050    PRINT #" CHECK DOSE SETTING"
2060    RETURN
2070    PRINT #"ERROR   ERROR   ERROR"
2080    RETURN
2090    PRINT #" REPEAT MEASUREMENT"
2100    RETURN
```

```
2110    REM ***
2120    REM ***
2130    REM ***
2140    REM TESTPG FOR HUDREFLEKTANSMETER.TEST.BAS
2150    REM *** INITIALISERING
2160    REM 100% refl=900 mV
2170    REM FFE7H=65511=CONTROLREG.
2180    REM FFE6H=65510= C-PORTE
2190    REM FFE5H=65509= B-PORTE.
2200    REM FFE4H=65508= A-PORTE.
2210    BAUD 2400 :   REM BAUDRATE TIL DISPLAY
2220   XBY(65511)=145 :   REM A INPUT,B OUTPUT,C-LOW INPUT,C-HI OUTPUT
2230    PRINT #CHR(27),"I",CHR(17),CHR(22),
2240    REM *** INIT SLUT
2250    PRINT #"TESTER LYDGIVER"
2260    FOR N=0 TO 1
2270   XBY(65509)=128
2280    FOR T=0 TO 50 :   NEXT T
2290   XBY(65509)=0
2300    FOR T=0 TO 50 :   NEXT T
2310    NEXT N
2320    PRINT #" TESTER OMSKIFTERE"
2330    DIM SW(6)
2340    FOR P=0 TO 5
2350   XBY(65509)=2**P
2360   SW(P)=XBY(65510).AND.15
2370    NEXT P
2380    FOR P=5 TO 4 STEP -1
2390    PRINT #SW(P),
2400    NEXT P
2410    PRINT #"   ",
2420    FOR P=3 TO 0 STEP -1
2430    PRINT #SW(P),
2440    NEXT P
2450    PRINT #
2460   SW2=1000*SW(3)+100*SW(2)+10*SW(1)+SW(0)
2470   SW1=10*SW(5)+SW(4)
2480    IF SW2=0 THEN 2500
2490    GOTO 2340
2500    PRINT #CHR(27),"I",CHR(17),CHR(22),
2510    PRINT #"  A/D-CONV.UDG. mV"
2520    GOSUB 2750
2530    PRINT #"  ",M, TAB (10),"          "
2540    PRINT #""
2550   XBY(65509)=1 : SW(0)=XBY(65510).AND.15
2560    IF SW(0)=1 THEN 2600
2570    IF SW(0)=2 THEN 2670
2580    GOTO 2520
2590    GOTO 2520
2600   XBY(65510)=16 :   REM PC4 HI GREEN DIODE ON.
2610    FOR T=0 TO 200 :   NEXT T
2620    GOSUB 2750
2630   XBY(65510)=0 :   REM  DIODES OFF
2640    PRINT #"  ",M, TAB (10),"GREEN    "
2650    PRINT #""
2660    GOTO 2550
```

```
2670  XBY(65510)=64 :   REM PC6 HI RED DIODE ON
2680   FOR T=0 TO 200 :   NEXT T
2690   GOSUB 2750
2700  XBY(65510)=0 :  REM   DIODES OFF
2710   PRINT #"   ",M, TAB (10),"RED        "
2720   PRINT #" "
2730   GOTO 2550
2740   REM ***AFLAESNING AD A/D-CONVERTER
2750   DO
2760  I=XBY(65508) : C=I.AND.112
2770   UNTIL C=112 :   REM I.E.PA6,PA5 OG PA4 HI.
2780   DO
2790  I=XBY(65508) : C=I.AND.112
2800   UNTIL C=48 :   REM   I.E. PA6 LO I.E. HUNDREDS SELECTED.
2810  H=I.AND.15
2820   DO
2830  I=XBY(65508) : C=I.AND.112
2840   UNTIL C=96 :  REM I.E. PA4 LO I.E. UNITS SELECTED.
2850  U=I.AND.15
2860   DO
2870  I=XBY(65508) : C=I.AND.112
2880   UNTIL C=80 :  REM I.E. PA5 L I.E. TENS SELECTED.
2890  T=I.AND.15
2900  FT=1
2910   IF H=10 THEN H=0 : FT=-1
2920  M=FT*(100*H+10*T+U)
2930   RETURN
2940   REM *** AFLAESNING SLUT
3000  LSW=1
3010   IF SW2=1 THEN SW2=334 :   REM INCL CORR 1.85
3020   IF SW2=2 THEN  GOTO 3200
3030   IF SW2=3 THEN   GOTO 3200
3040   IF SW2=4 THEN   GOTO 3200
3050   IF SW2=5 THEN   GOTO 3200
3060   IF SW2=6 THEN   GOTO 3200
3070   IF SW2=7 THEN GOTO 3200
3080   IF SW2=8 THEN SW2=52880
3090   IF SW2=9 THEN SW2=21081
3100   IF SW2=10 THEN SW2=52880
3110   IF SW2=11 THEN GOTO 3200
3120   IF SW2=12 THEN SW2=82
3130   IF SW2=13 THEN GOTO 3200
3140   IF SW2=14 THEN goto 120
3150   IF SW2=15 THEN SW15=1
3160   GOTO 1490
3200   GOSUB 2010
3210   GOSUB 2070
3220   FOR T=0 TO 2000 :   NEXT T
3230   GOTO 90
```

In case the programme is adapted to control the microprocessor of the apparatus according to the present invention in accordance with the above described alternative calculations relating to the comparison of the UV dose in B-MED and the degree of pigmentation, the lines 1500 and 1510 of the programme are amended into:

```
1500   TT=(-0.206+0.689xORD+0.0829xPIG%) and
1510   TT=.689x(ORD-ARD).
```

The apparatus according to the present invention should be operated in accordance with the below measuring guidelines:

The undressed patient should acclimate before the measurement. This is to evaporate sweat from the skin and cool. Measurements is performed on normal or relatively normal skin avoiding discoloration from treatment, freckles, hairy areas even after shaving, flushing zones, and skin irritated from rubbing by the person himself or from the back of a chair. If fullbody treatment is performed the best place to measure is on the back above a horizontal line through the lower part of the scapulae, and on the upper part of the abdomen and the breast. Measurements on the extremities will only be reproducible if the patient is placed horizontally and relaxing. Redness will change with level and activity.

The detector is held perpendicular to the surface of the skin gently touching the skin in order to eliminate light from entering the detector from the environment.

Light sources used for the treatment of skin diseases may have very different biological activity. And since the limiting factor for the treatment dose is redness of the skin, the use of a basic MED has been chosen as the basic dose. The basic MED being calculated from the erythema action spectrum CIE (McKinlay & Diffey) and the MED dose of 31,2 mJ/cm2 at 296 nm (Parrish).

The correct number of seconds to reach that dose, i.e. 1B-MED, is entered into the microprocessor by means of the the thumb wheel 20 at the right-hand side of the display 16.

By means of the thumb wheel 16 at the left-hand side of the display 20, the "24h erythema level" is input. This level has to be decided by the person operating the apparatus.

0.0     Corresponds to the highest dose which can be given before redness will appear.

0.5     Corresponds to (+) redness, weak spotted redness without sharp borders to the untreated surroundings.

1.0     Weak redness with a clear demarcation to the surroundings. Often named +.

2.0     Corresponds to redness ++ with clear redness and a weak edema to be felt.

3.0     Corresponds to +++ redness. There is heavy redness with edema above the surroundings.

Therefore, the following schedule is recommended for the treatment of psoriasis:

| 1. Week of treatment | setting 1.0 |
|---|---|
| 2. Week of treatment | 3.0 |
| 3. week of treatment | 6.0 |
| 4. Week of treatment | 9.0 |

A constant erythema level setting may be used for atopic dermatitis mycosis fungiodes, pruritus etc.

Pigmentation

The scale has been chosen so that people with a very wide range of pigmentation covering all races are represented in the scale. Pigmentation is given on a scale from 0% to 100%. 0% pigmentation represents the degree of pigmentation of previously unirradiated buttocks of an extremely white person. 100% pigmentation represents the degree of pigmentation of previously unirradiated buttocks of a very black person.

In the first few days after intensive UV irradiation redness corresponding to ++ and +++ etc. will result in a lower measure of pigmentation than the measurement preceding irradiation. This phenomenon is a result of edema following heavy erythema and is caused by erythema itself.

Erythema

The scale is chosen so that people with zero blood flow and dark blue red naevus flammeus can be fitted into the scale.

0% redness has been chosen as the redness of the skin on the antebrachium of persons who had their arm emptied for blood and the blood supply stopped by a cuff on the upper arm.

100% has been chosen so that even very blue-red naevus flammeus of the face may be included.

0-30% corresponds to what is found under normal conditions in connection with people acclimated to room temperature.

The relations between the clinical degree of redness and the average % of UV irradiated people or individuals are listed in Table 1.

The pigment protection factor indicates how much higher UV dose an individual can take before obtaining + in redness compared to a person reacting with + redness.

After exposure to 1 B-MED from a Philips TL 12 equipped radiation source. All background measurements are to be performed on previously unexposed buttocks.

Treatment time shown on the display 16 is the time used for full body irradiation. Therefore, the irradiation dose matches the most sensitive larger areas of the body. It should be noted that the treatment time depends on the radiation source and the setting of the thumb wheel switch 20.

When the thumb wheel switch 20 at the right-hand side of the display 16 is adjusted to 0000 (no radiation) the apparatus automatically jumps into calibration mode.

Keeping the detector close to the tile which is delivered together with the apparatus a reflection % for the red and green light is shown on the display. This reflection should be within the limits written on the tile.

Before calibration, the apparatus must be in temperature equilibrium with the room in which the apparatus is to be used.

The calibration values is traceable to standard ISO 2469. Using this standard will display 100% both for red and green reflection.

The detector is constructed to demand a minimum of cleaning. Only a plastic ring is in contact with the skin. This may be cleaned by an alcoholic solution.

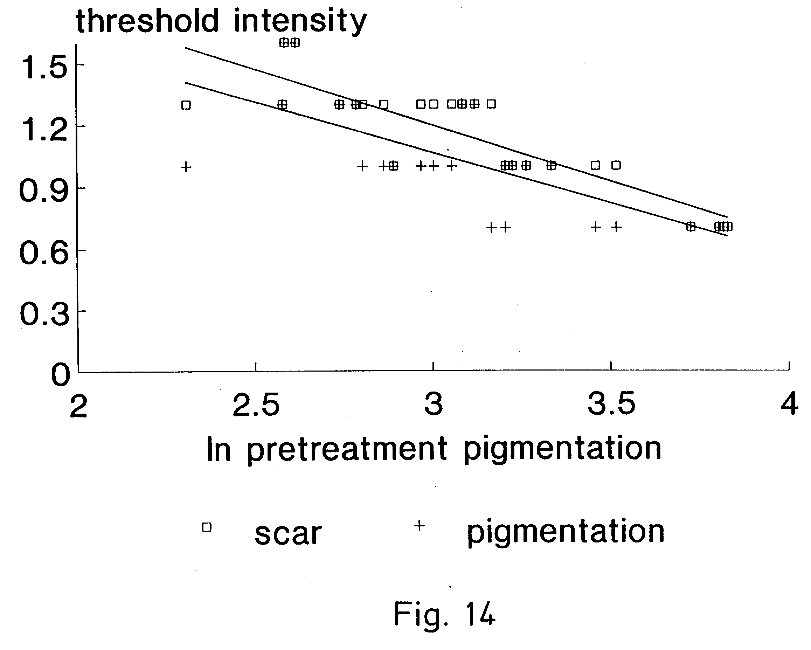

In Fig. 14 a diagram is shown illustrating the basic realization that a linear relationship exists between light- or laser-induced skin changes to individuals and the degree of skin pigmentation of the same individuals. The diagram shown in Fig. 14 was based on the below test.

Thirteen individuals with a varying degree of epidermal skin pigmentation, objectified by skin reflectance, were on the inside of the upper arm treated with a total of six hexagonal areas by an argon laser (AL, 488 nm) and a copper vapor laser (CVL, 578nm). The lasers were connected to a Hexascan device, and physical parameters were identical for the two laser types, except for the wavelengths. Beam diameter was 1 mm, pulse duration 200 msec, intensities 0.7, 1.0, and 1.3 W/spot, resulting in the following spot doses 17.8, 25.5, and 33.1 J/cm$^2$.

A correlation was demonstrated between pre-treatment skin pigmentation and the clinical effects obtained immediately after and 6 months after the laser treatment. Assessment of the chronic response was based on distinction between pigmentary changes and scarring.

The CVL induced a significantly higher degree of acute results at the 0.7 and 1.0 W/spot treatment level as compared with the AL. For the clinical response 6 months after the laser treatment it turned out that the AL at the 1.0 and 1.3 W/spot induced a significantly higher degree of hyperpigmentation and scarring as compared with the CVL.

An inverse relation was found between the degree of pre-treatment pigmentation and the threshold intensities required to induce wound formation, scar formation and postinflammatory hyperpigmentation, respectively.

Detailed measuring results obtained by the above test are illustrated in the diagrams 15A-15L.

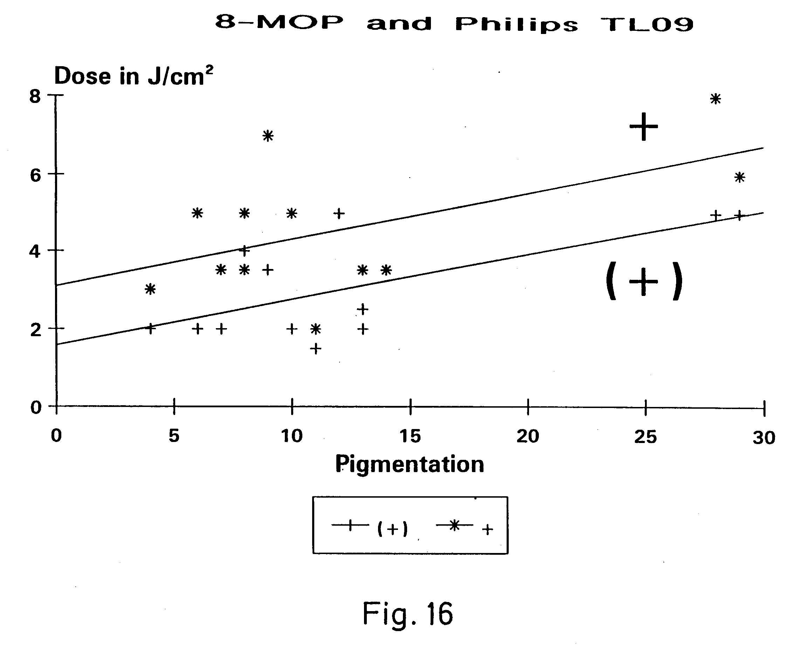

In Fig. 16, a diagram is shown, illustrating the relation of using the degree of skin pigmentation as a predictor of minimal phototoxic dose to be given to the same individual. The diagram represents the result of the following test or experiment.

Fourteen individuals with skin type (II) (11) and skin type III (3) participated in the test. The sensitivity to irradiation with Philips TL12 tubes was determined without medication on previous unexposed buttocks (MED). The sensitivity to irradiation with Philips TL09 tubes was determined 1 h after oral administration of (0.44-0.63 mg/kg) 8-MOP in a similar way (MPD). The pigmentation of the test areas before irradiation was quantitated with an apparatus according to the present invention, the erythema reaction was assessed clinically using a (+)-+++ scale 24 h after TL12, and 72h after TL09 exposure.

The pigmentation % of the unexposed test areas vs. the energy needed to elicit MED or MPD was plotted in scattergrams. There was a positive relation between pigmentation and the dose of TL12 needed to elicit a (+) or + erythemal reaction. The lines of the (+) and + reaction had identical slopes. Similar results were observed with TL09 exposure after ingestion of 8-MOP. Spearmanns test for correlation between pigmentation and dose to erythemal reaction was significant for the + reaction after TL12 exposure, $p<0.05$. The correlation coefficient for the other parameters was non-significant ($0.4>p>0.05$).

A further experiment was made:

Seventeen volunteers were tested with Philips TL12 and TL01 tubes on previous unexposed buttocks. Biological doses ranging from 0.25 to 3.00 B-MEDs were used.

B-MED derived from the erythema action spectrum of McKinlay and Diffey and 1 MED = 312 J/m$^2$ at 296 nm (Par-

rish). The physical doses corresponding to 1 B-MED are 0.62 J/m$^2$ for the TL01 tube and 0.082 J/m$^2$ for the TL12 tube (TL12:TL01 ~ 1:7.5).

The redness % and pigment protection factor (PPF) were measured before and 24 h after exposure with an apparatus according to the present invention. Also the test sites were evaluated clinically after 24 h. Test sites with redness %>30 measured by the apparatus or clinical redness ((+)-+++) were included in the study. The results were given as redness % as a function of dose. Since a linear reciprocity between PPF before exposure and dose to give a specific effect is assumed to exist, the results were also given as redness % as a function of dose/PPF. As the intercept of the four lines did not differ significantly, a common intercept of 22.5 was used.

Since identical biological doses were given of the TL12 and TL01, an identical redness was expected. However, it was found that the dose ratio between TL12 and TL01 to obtain the same redness values was 1:0.55, the TL01 being 1.85 times stronger than TL12. The results are in accordance with the assumption that a linear reciprocity between PPF and the dose needed to produce a specific response exists, since it was found that 1 MED/PPF from the TL12 tube resulted in a redness of 37.1%, not significantly different from the expected 36.9%, the value corresponding to the clinical reading +.

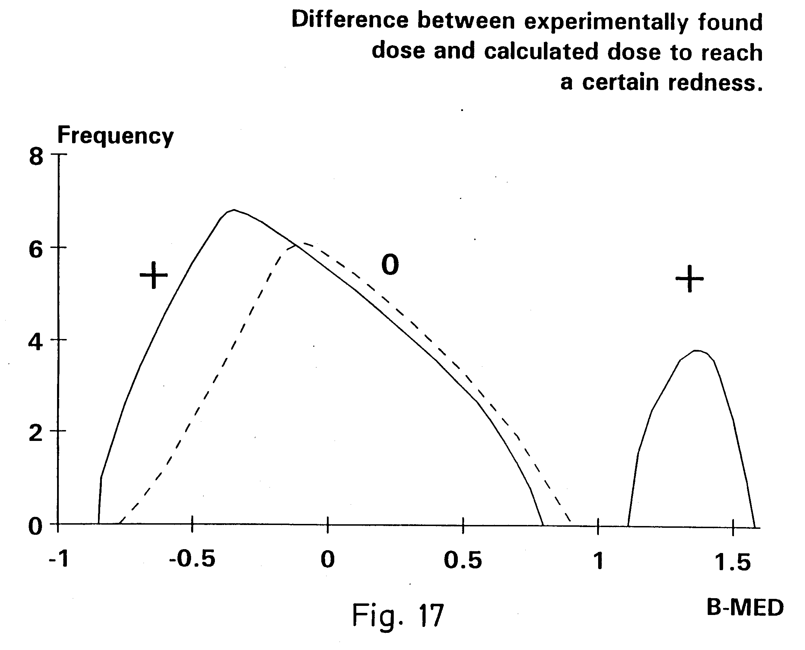

In Fig. 17, a diagram is shown illustrating the correspondence or the difference between experimentally found UV doses and doses calculated to reach a certain redness. The diagram is based on measuring results obtained from the same 49 individuals investigated and used for the experiments or tests on basis of which the diagram shown in Fig. 6 is derived.

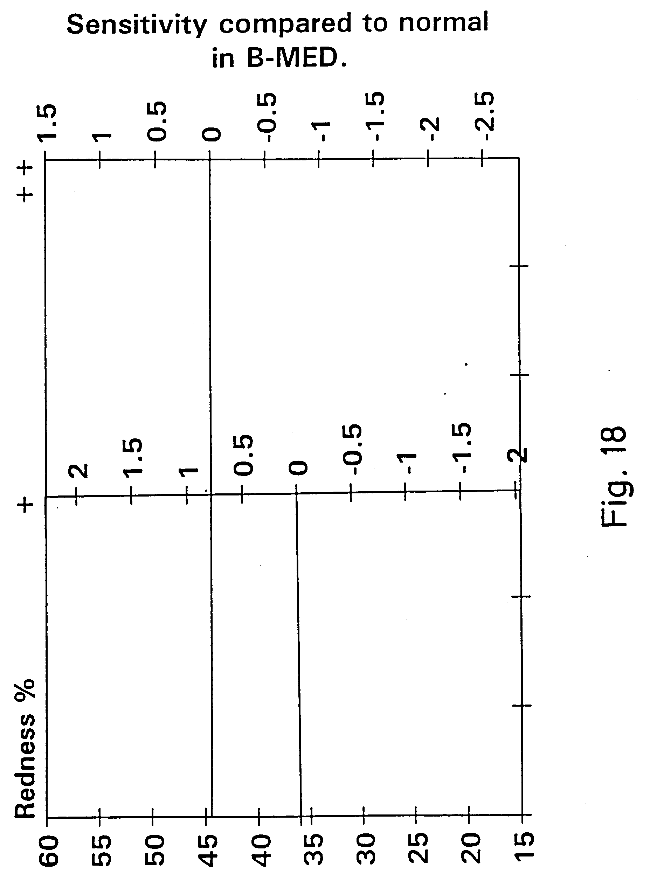

In Fig. 18, a diagram is shown, which diagram illustrates the adaptation of the present invention as to determining the sensitivity of an individual who has been exposed to UV treatment. The individual has after determination of the individual's ability to stand exposure to UV radiation, by employing the method and the apparatus according to the present invention, been exposed to UV treatment, i.e. been exposed to a specific UV dose. After the UV treatment, the redness of the individual is as discussed above with reference to Fig. 8 determined. The redness % is converted into a B-MED measure or difference at + redness and ++ redness, which B-MED measure represents the difference between the UV dose to which the individual has been exposed, and the UV dose which the individual may stand.

## DEFINITIONS

B-MED     Basic Minimal Erythema Dose is 312 J/m$^2$ at 296 nm. ((24 h erythema) Parrish).

MED     Minimal Erythema Dose for an individual 24 h after exposure.

PPF     PPF x B-MED = MED

        The Pigment Protection Factor is the number of B-MEDs which elicit + redness in an individual 24 h after exposure.

Erythema Action Spectrum: CIE (McKinlay & Diffey).

## Claims

1. A method of determining an individual's ability to become tanned or to stand exposure to ultraviolet radiation without causing a skin reaction, such as skin cancer or erythema, comprising the following steps:

    exposing at least part of said individual's skin surface to electromagnetic radiation of a first wavelength and of a predetermined intensity, said first wavelength being a wavelength at which erythrodermic skin reflection is high,
    measuring the intensity of electromagnetic radiation reflected from said part of said individual's skin surface so as to determine a first coefficient of reflection of said skin surface part to said electromagnetic radiation of said first wavelength,
    exposing said skin surface part to electromagnetic radiation of a second wavelength and of a predetermined intensity, said second wavelength being a wavelength at which erythrodermic skin reflection is low, and
    measuring the intensity of electromagnetic radiation reflected from said part of said individual's skin surface part so as to determine a second coefficient of reflection of said skin surface part to electromagnetic radiation of said second wavelength, characterized by
    comparing said first and second coefficients of reflection with predetermined sets of coefficients of reflection of said first and second wavelengths representing coherent sets of coefficients of reflection of said first and second wavelengths of respective, specific states of redness so as to determine said individual's skin surface part's state of redness,

converting said first and second coefficients of reflection into a specific set of corrected first and second coefficients of reflection of a specific state of redness, so as to determine said individual's skin surface part's coefficients of reflection of said first and second wavelengths at said specific state of redness, and

converting said specific set of corrected first and second coefficients of reflection into a measure representing said individual's ability to become tanned or to stand exposure to ultraviolet radiation without causing said skin reaction.

2. A method according to Claim 1, further comprising the step of determining said individual's skin surface part's degree of pigmentation from said corrected first and second coefficients of reflection of said first and second wavelengths at said specific state of redness.

3. A method according to Claims 1 and 2, said specific state of redness corresponding to an average zero blood flow state.

4. A method according to any of the Claims 1-3, further comprising the steps of:

converting said second coefficient of reflection into logarithmic representation, and said sets of coefficients of reflection representing coherent sets of coefficients of reflections of said first and second wavelengths comprising coefficients of reflection of said second wavelengths presented in logarithmic representation.

5. An apparatus for determining an individual's ability to become tanned or to stand exposure to ultraviolet radiation without causing a skin reaction, such as skin cancer or erythema, comprising:

a first electromagnetic source for generating electromagnetic radiation of a first wavelength and of a predetermined intensity and for directing said electromagnetic radiation of said first wavelength to a part of said individual's skin surface so as to expose said part of said individual's skin surface to said electromagnetic radiation of said first wavelength,

a second electromagnetic source for generating electromagnetic radiation of a second wavelength and of a predetermined intensity and for directing said electromagnetic radiation of said second wavelength to said part of said individual's skin surface so as to expose said part of said individual's skin surface to said electromagnetic radiation of said second wavelength,

a light-detecting means for measuring the intensity of electromagnetic radiation reflected from said part of said individual's skin surface, and

a measuring means connected to said light-detecting means for measuring the intensity of electromagnetic radiation reflected from said part of said individual's skin surface so as to determine a first and a second coefficient of reflection of said skin surface part to said electromagnetic radiation of said first and second wavelength, respectively, characterized by

a comparison and converting means connected to said measuring means for comparing said first and second coefficients of reflection with predetermined sets of coefficients of reflection of said first and second wavelengths representing coherent sets of coefficients of reflection of said first and second wavelengths of respective, specific states of redness so as to determine said individual's skin surface part's state of redness, for converting said first and second coefficients of reflection into a specific set of corrected first and second coefficients of reflection of a specific state of redness, so as to determine said individual's skin surface part's coefficients of reflection of said first and second wavelengths at said specific state of redness, and for converting said specific set of corrected first and second coefficients of reflection into a measure representing said individual's ability to become tanned or to stand exposure to ultraviolet radiation without causing said skin reaction.

6. An apparatus according to Claim 5, said light-detecting means comprising separate first and second light detector means for detecting electromagnetic radiation of said first and second wavelengths, respectively, reflected from said part of said individual's skin surface part.

7. An apparatus according to any of the Claims 5-6, said comparison and converting means further determining said individual's skin surface part's degree of pigmentation from said corrected first and second coefficients of reflection of said first and second wavelengths at said specific state of redness.

8. An apparatus according to any of the Claims 5-7, said specific state of redness being an average Zero blood flow state.

9. An apparatus according to any of the Claims 5-8, said comparison and converting means further converting said second coefficient of reflection into logarithmic representation, and said sets of coefficients of reflection representing coherent sets of coefficients of reflection of said first and second wavelengths comprising coefficients of reflection of said second wavelengths represented in logarithmic representation.

10. An apparatus according to any of the Claims 5-9, said first wavelength being of the order of 660 nm, and said second wavelength being of the order of 550 nm.

**Patentansprüche**

1. Verfahren zur Bestimmung der Fähigkeit von Personen, braun zu werden oder eine Belichtung mit ultravioletter Strahlung auszuhalten, ohne daß eine Hautreaktion hervorgerufen wird, wie beispielsweise Hautkrebs oder Erythem, wobei das Verfahren die folgenden Schritte aufweist:

Belichtung von zumindest einem Teil der Hautoberfläche einer Person mit einer elektromagnetischen Strahlung mit einer ersten Wellenlänge und mit einer vorgegebenen Intensität, wobei die erste Wellenlänge eine Wellenlänge ist, bei der die erythrodermische Hautreflektion hoch ist,

Messung der Intensität der von dem Teil der Hautoberfläche der Person reflektierten elektromagnetischen Strahlung, um einen ersten Reflektionskoeffizienten des Teils der Hautoberfläche in Bezug auf die elektromagnetische Strahlung mit der ersten Wellenlänge zu bestimmen,

Belichtung des Teils der Hautoberfläche mit einer elektromagnetischen Strahlung mit einer zweiten Wellenlänge und mit einer vorgegebenen Intensität, wobei die zweite Wellenlänge eine Wellenlänge ist, bei der die erythrodermische Hautreflektion gering ist, und

Messung der Intensität der von dem Teil der Hautoberfläche der Person reflektierten elektromagnetischen Strahlung, um einen zweiten Reflektionskoeffizienten des Teils der Hautoberfläche in Bezug auf die elektromagnetische Strahlung mit der zweiten Wellenlänge zu bestimmen,
**gekennzeichnet durch**

Vergleichen des ersten und des zweiten Reflektionskoeffizienten mit einem vorgegebenen Satz von Reflektionskoeffizienten für die erste und die zweite Wellenlänge, die einen kohärenten Satz von Reflektionskoeffizienten der ersten und zweiten Wellenlänge von entsprechenden spezifischen Rötungszuständen darstellen, um somit den Rötungszustand des Teils der Hautoberfläche der Person zu bestimmen,

Konvertieren des ersten und zweiten Reflektionskoeffizienten in einen spezifischen Satz von korrigierten ersten und zweiten Reflektionskoeffizienten eines spezifischen Rötungszustandes, um die Reflektionskoeffizienten des Teils der Hautoberfläche der Person für die erste und die zweite Wellenlänge in einem spezifischen Rötungszustand zu bestimmen, und

Konvertieren des spezifischen Satzes von korrigierten ersten und zweiten Reflektionskoeffizienten in einen Wert, der die Eigenschaft der Person darstellt, braun zu werden oder einer Belichtung mit ultravioletter Strahlung Stand zu halten, ohne daß eine Hautreaktion hervorgerufen wird.

2. Verfahren nach Anspruch 1, das weiter den Schritt des Bestimmens des Grades der Pigmentierung des Teils der Hautoberfläche der Person aus den korrigierten ersten und zweiten Reflektionskoeffizienten für die erste und die zweite Wellenlänge in dem spezifischen Rötungszustand aufweist.

3. Verfahren nach Anspruch 1 und 2, wobei der spezifische Rötungszustand mit einem Zustand bei im Durchschnitt Null Blutfluß entspricht.

4. Verfahren nach einem der Ansprüche 1 bis 3, das weiterhin die Schritte aufweist:

Konvertieren des zweiten Reflektionskoeffizienten in einer logarythmischen Darstellung und Darstellen der Sätze der Reflektionskoeffizienten, die die kohärenten Sätze von Reflektionskoeffizienten für die erste und die zweite Wellenlänge repräsentieren, die die Reflektionskoeffizienten für die zweite Wellenlänge enthalten, in einer logarythmischen Darstellung.

5. Vorrichtung zur Bestimmung der Fähigkeit einer Person, braun zu werden oder einer Belichtung mit ultravioletter Strahlung Stand zu halten, ohne daß eine Hautreaktion erzeugt wird, wie beispielsweise Hautkrebs oder Erythem, wobei die Vorrichtung aufweist:

eine erste elektromagnetische Quelle zur Erzeugung von elektromagnetischer Strahlung mit einer ersten Wellenlänge und mit einer vorbestimmten Intensität und zur Ausrichtung der elektromagnetischen Strahlung der ersten Wellenlänge auf einen Teil der Hautoberfläche der Person, so daß der Teil der Hautoberfläche der Person mit der elektromagnetischen Strahlung mit der ersten Wellenlänge belichtet wird,

eine zweite elektromagnetische Quelle zur Erzeugung von elektromagnetischer Strahlung mit einer zweiten Wellenlänge und mit einer vorbestimmten Intensität und zur Ausrichtung der elektromagnetischen Strahlung mit der zweiten Wellenlänge auf den Teil der Hautoberfläche der Person, so daß der Teil der Hautoberfläche der Person mit der elektromagnetischen Strahlung der zweiten Wellenlänge belichtet wird,

ein Licht nachweisendes Mittel zum Messen der Intensität der elektromagnetischen Strahlung, die von dem Teil der Hautoberfläche der Person reflektiert wird, und

eine Meßvorrichtung, die mit dem Licht nachweisenden Mittel zur Messung der Intensität der elektromagnetischen Strahlung, die von dem Teil der Hautoberfläche der Person reflektiert wird, verbunden ist, um einen ersten und einen zweiten Reflektionskoeffizienten des Teils der Hautoberfläche bezüglich der elektromagnetischen Strahlung der ersten und der zweiten Wellenlänge zu bestimmen,
**gekennzeichnet durch**

eine Vergleichs- und Konvertierungsvorrichtung, die mit der Meßvorrichtung verbunden ist, um die ersten und zweiten Reflektionskoeffizienten mit einem vorbestimmten Satz von Reflektionskoeffizienten für die erste und die zweite Wellenlänge zu vergleichen, die einen kohärenten Satz von Reflektionskoeffizienten der ersten und zweiten Wellenlänge von entsprechenden spezifischen Rötungszuständen darstellen, so daß der Rötungszustand des Teils der Hautoberfläche der Person bestimmt wird, zum Konvertieren der ersten und der zweiten Reflektionskoeffizienten in einen spezifischen Satz von korrigierten ersten und zweiten Reflektionskoeffizienten für einen spezifischen Rötungszustand, so daß die Reflektionskoeffizienten des Teils der Hautoberfläche der Person für die erste und die zweite Wellenlänge bei dem spezifischen Rötungszustand bestimmt wird, und zum Konvertieren des spezifischen Satzes von korrigierten ersten und zweiten Reflektionskoeffizienten in einen Meßwert, der die Fähigkeit der Person repräsentiert, braun zu werden oder einer Belichtung mit ultravioletter Strahlung Stand zu halten, ohne daß eine Hautreaktion hervorgerufen wird.

6. Vorrichtung nach Anspruch 5, wobei das Licht nachweisende Mittel separate erste und zweite Licht nachweisende Mittel zum Nachweis elektromagnetischer Strahlung mit der ersten bzw. der zweiten Wellenlänge, die von dem Teil der Hautoberfläche der Person reflektiert wird, aufweist.

7. Vorrichtung nach einem der Ansprüche 5 bis 6, wobei die Vergleichs- und Konvertierungsvorrichtung weiterhin den Grad der Pigmentierung des Teils der Hautoberfläche der Person aus den korrigierten ersten und zweiten Reflektionskoeffizienten für die erste und die zweite Wellenlänge bei dem spezifischen Rötungszustand bestimmt.

8. Vorrichtung nach einem der Ansprüche 5 bis 7, wobei der spezifische Rötungszustand ein Zustand bei im Durchschnitt Null Blutfluß ist.

9. Vorrichtung nach einem der Ansprüche 5 bis 8, wobei die Vergleichs- und Konvertierungsvorrichtung weiterhin den zweiten Reflektionskoeffizienten in eine logarythmische Darstellung konvertiert und wobei der Satz der Reflektionskoeffizienten, die einen kohärenten Satz von Reflektionskoeffizienten für die erste und die zweite Wellenlänge darstellt, Reflektionskoeffizienten für die zweite Wellenlänge in einer logarythmischen Darstellung aufweist.

10. Vorrichtung nach einem der Ansprüche 5 bis 9, wobei die erste Wellenlänge im Bereich von 660 nm und die zweite Wellenlänge im Bereich von 550 nm liegt.

**Revendications**

1. Procédé de détermination d'une capacité d'un individu à devenir bronzé ou à supporter l'exposition à du rayonnement ultraviolet sans que n'ait lieu une réaction de la peau, telle qu'un cancer de la peau ou un érythème, qui comprend les étapes suivantes :

on expose au moins une partie de la surface de la peau de l'individu à du rayonnement électromagnétique d'une première longueur d'onde et d'une intensité déterminée à l'avance, la première longueur d'onde étant une longueur d'onde à laquelle la réflexion érythrodermique de la peau est élevée,

on mesure l'intensité de rayonnement électromagnétique réfléchi à partir de ladite partie de la surface de la peau de l'individu de manière à déterminer un premier coefficient de réflexion de cette partie de surface de la peau au rayonnement électromagnétique à la première longueur d'onde,

on expose la partie de surface de la peau à du rayonnement électromagnétique d'une seconde longueur d'onde et d'une intensité déterminée à l'avance, la seconde longueur d'onde étant une longueur d'onde à laquelle la réflexion érythrodermique de la peau est faible, et

on mesure l'intensité du rayonnement électromagnétique réfléchi par cette partie de la surface de la peau de l'individu de manière à déterminer un second coefficient de réflexion de cette partie de la surface de la peau au rayonnement électromagnétique à la seconde longueur d'onde, caractérisé par le fait qu'

on compare les premier et second coefficients de réflexion à des ensembles déterminés à l'avance de coefficients de réflexion aux première et seconde longueurs d'onde représentant des ensembles cohérents de coefficients de réflexion aux première et seconde longueurs d'onde d'états précis respectifs de rougeur de manière à déterminer l'état de rougeur de la partie de la surface de la peau de l'individu,

on convertit les premier et second coefficients de réflexion en un ensemble précis de premier et second coefficients de réflexion corrigés d'un état de rougeur précis, de manière à déterminer les coefficients de réflexion de la partie de la surface de la peau de l'individu aux première et seconde longueurs d'onde à l'état précis de rougeur, et

on convertit l'ensemble précis des premier et second coefficients corrigés de réflexion en une mesure représentant la capacité de l'individu à devenir bronzé ou à supporter l'exposition à du rayonnement ultraviolet sans entraîner de réaction de la peau.

2. Procédé suivant la revendication 1, comportant en outre l'étape qui consiste à déterminer le degré de pigmentation de la partie de la surface de la peau de l'individu à partir des premier et second coefficients de réflexion corrigés aux première et seconde longueurs d'onde à l'état de rougeur précis.

3. Procédé suivant la revendication 1 et 2, l'état précis de rougeur correspondant à un état de débit sanguin moyen nul.

4. Procédé suivant l'une quelconque des revendications 1 à 3, comportant en outre les étapes qui consistent à :

convertir le second coefficient de réflexion en une représentation logarithmique, et les ensembles des coefficients de réflexion représentant des ensembles cohérents de coefficients de réflexion aux première et seconde longueurs d'onde comportant des coefficients de réflexion aux secondes longueurs d'onde présentés suivant une représentation logarithmique.

5. Dispositif destiné à déterminer une capacité d'un individu à devenir bronzé ou à supporter l'exposition à du rayonnement ultraviolet sans entraîner une réaction de la peau telle qu'un cancer de la peau ou un érythème, comportant :

une première source électromagnétique destinée à produire du rayonnement électromagnétique à une première longueur d'onde et à une intensité déterminé à l'avance et destinée à diriger le rayonnement électromagnétique à la première longueur d'onde vers une partie de la surface de la peau de l'individu de manière à exposer cette partie de la surface de la peau de l'individu au rayonnement électromagnétique à la première longueur d'onde,

une seconde source électromagnétique destinée à produire du rayonnement électromagnétique à une seconde longueur d'onde et à une intensité déterminée à l'avance et destinée à diriger le rayonnement électromagnétique à la seconde longueur d'onde vers la partie de la surface de la peau de l'individu de manière à exposer cette partie de la surface de la peau de l'individu au rayonnement électromagnétique à la seconde longueur d'onde, des moyens détecteurs de lumière destinés à mesurer l'intensité du rayonnement électromagnétique réfléchi par cette partie de la surface de la peau de l'individu, et

des moyens de mesure reliés aux moyens de détection de lumière destinés à mesurer l'intensité du rayonnement électromagnétique réfléchi par cette partie de la surface de la peau de l'individu de manière à déterminer un premier et un second coefficient de réflexion de la partie de la surface de la peau au rayonnement électromagnétique aux première et seconde longueurs d'onde respectivement, caractérisé par

des moyens de comparaison et de conversion reliés aux moyens de mesure destinés à comparer les premier et second coefficients de réflexion à des ensembles déterminés à l'avance de coefficients de réflexion aux première et seconde longueurs d'onde représentant des ensembles cohérents de coefficients de réflexion aux première et seconde longueurs d'onde d'états de rougeur respectifs précis de manière à déterminer l'état de rougeur de cette partie de la surface de la peau de l'individu, destinés à convertir les premier et second coefficients de réflexion en un ensemble précis de premier et second coefficients de réflexion corrigés de l'état de rougeur précis, de manière à déterminer les coefficients de réflexion de la partie de la surface de la peau de l'individu aux première et seconde longueurs d'onde à l'état de rougeur précis, et destinés à convertir l'ensemble précis de premier et second coefficients de réflexion corrigés en une mesure représentant la capacité de l'individu à devenir bronzé ou à supporter l'exposition à du rayonnement ultraviolet sans entraîner de réaction de la peau.

6. Dispositif suivant la revendication 5, les moyens de détection de lumière comportant des premier et second moyens détecteurs de lumière distincts destinés à détecter du rayonnement électromagnétique à la première et seconde longueur d'onde respectivement, réfléchi à partir de la partie de la surface de la peau de l'individu.

7. Dispositif suivant l'une quelconque des revendications 5 à 6, les moyens de comparaison et de conversion déterminant en outre le degré de pigmentation de la partie de la surface de la peau de l'individu à partir des premier et second coefficients de réflexion corrigés aux première et seconde longueurs d'onde à l'état de rougeur précis.

8. Dispositif suivant l'une quelconque des revendications 5 à 7, l'état de rougeur précis étant un état de débit sanguin moyen nul.

9. Dispositif suivant l'une quelconque des revendications 5 à 8, les moyens de conversion et de comparaison convertissant en outre le second coefficient de réflexion en une représentation logarithmique et les ensembles de coefficients de réflexion représentant des ensembles cohérents de coefficients de réflexion aux première et seconde longueurs d'onde comportant des coefficients de réflexion à la seconde longueur d'onde représentés suivant la représentation logarithmique.

10. Dispositif suivant l'une quelconque des revendications 5 à 9, la première longueur d'onde étant de l'ordre de 660 nm, et la seconde longueur d'onde étant de l'ordre de 550 nm.

# Fig. 1

A : White skin
B : A with redness

# Fig. 2

A : White skin
C : Pigmented skin

EP 0 629 124 B1

Fig. 3

EP 0 629 124 B1

# Fig. 4

EP 0 629 124 B1

Fig. 5A

Fig. 5B

Fig. 5C

EP 0 629 124 B1

VOLAR SIDE OF ANTEBRACHIUM

0-FLOW

A
B
C

D

Ln (GREEN REFLECTION %)

A   AVERAGE 0-FLOW
B   VERTICAL
C   HORIZONTAL
D   REACTIVE REDNESS

Fig. 8

Fig. 9A

+++ REDNESS

++ REDNESS

+ REDNESS

0−REACTION

BASIC MED

10

5

0

3    4    5

Ln (CORRECTED RED REFLECTION %)

EP 0 629 124 B1

**Fig. 9B**

Fig. 10

Fig. 11

EP 0 629 124 B1

Fig. 12 A

Fig. 12 B

43

# Fig. 13

threshold intensity

In pretreatment pigmentation

□ scar     + pigmentation

Fig. 14

Fig. 15A
Fig. 15B
Fig. 15C

EP 0 629 124 B1

Fig. 15E

max. wound area

1.0 W

pigmentation

Fig. 15D

max. wound area

0.7 W

pigmentation

Fig. 15F

max. wound area

1.3 W

pigmentation

47

# Fig. 15 G

# Fig. 15 H

# Fig. 15 I

EP 0 629 124 B1

Fig. 15J

Fig. 15K

Fig. 15L

8-MOP and Philips TL09

Dose in J/cm²

Pigmentation

(+) ·····*····· +

Fig. 16

EP 0 629 124 B1

**Difference between experimentally found dose and calculated dose to reach a certain redness.**

Fig. 17

EP 0 629 124 B1

Fig. 18

EP 0 629 124 B1